# FASCICULE DE BREVET EUROPEEN

(11) **EP 2 254 687 B1**
(45) Date de publication et mention de la délivrance du brevet: **09.05.2012**
(21) Numéro de dépôt: 09722468.7
(22) Date de dépôt: 18.03.2009
(51) Int. Cl.: B01F 3/04, B01F 7/00, B01F 7/16, B01F 7/18, B01F 7/22, B01F 13/08, B01F 15/00, B01F 15/06, C12M 1/00, C12M 1/06

(54) **RECIPIENT-MELANGEUR**
MISCHGEFÄSS
MIXING VESSEL

(30) Priorité: 19.03.2008 US 69977 P
(43) Date de publication de la demande: 01.12.2010
(73) Titulaire: Sartorius Stedim Biotech GmbH, 37079 Göttingen (DE)
(72) Inventeur: LUDWIG, Jens, D-37127 Jühnde (DE); REIF, Oscar-Werner, D-30173 Hannover (DE); GRELLER, Gerhard, D-37085 Göttingen (DE); KAHLERT, Wolfgang, D-34327 Körle (DE); PRADEL, Günther, D-37077 Göttingen (DE); BATES, Michael, Gloucester Gloucestershire GL3 4PD (GB); BARBAROUX, Magali, 13112 La Destrousse (FR); BAUD, Stéphane, 13720 La Bouilladisse (FR); GAY, Isabelle, 13124 Peypin (FR); CHAUSSIN, Sébastien, 13400 Aubagne (FR)
(74) Mandataire: Derambure, Christian
(86) Numéro de dépôt international: PCT/IB2009/051150
(87) Numéro de publication internationale: WO 2009/116002

(56) Documents cités:
- EP-A- 1 884 561
- EP-A- 2 039 754
- EP-A- 2 065 085
- WO-A-2008/088371
- GB-A- 1 519 526
- US-A- 3 625 834
- US-A- 4 670 397
- US-A- 4 960 706
- US-A- 5 795 732

## Description

L'invention est relative au domaine des récipients-mélangeurs.

Elle vise plus particulièrement un récipient-mélangeur destiné à recevoir un contenu biopharmaceutique en vue de son mélange, un tel récipient-mélangeur faisant fonction de bioréacteur et un procédé de mise en oeuvre d'un tel récipient-mélangeur.

Des réservoirs de mélange ou avec agitateur comportant un appareil d'agitation rotatif sont généralement utilisés pour mélanger les composés chimiques. Les ingrédients mélangés dans les réservoirs avec agitateur exigent fréquemment un environnement stérile, tel que lorsque les ingrédients sont mélangés pour préparer un produit pharmaceutique. Bien que certaines applications n'exigent pas d'environnement stérile, la Food and Drug Administration des USA a publié des exigences strictes de stérilité pour certaines solutions Pour assurer un environnement stérile de ce type, les réservoirs de mélange doivent être construits pour éviter l'entrée de contaminants à l'intérieur pendant l'ensemble du processus discontinu, y compris pendant le remplissage, le mélange et la vidange des réservoirs.

L'utilisation d'entraînements magnétiques pour entraîner l'appareil d'agitation est devenue plus courante puisque ces entraînements n'exigent pas de raccordement physique ou de joints entre les parties mobiles du moyen d'entraînement et de l'appareil d'agitation dans l'environnement stérile. Dans les réservoirs avec agitateur adaptés à l'appareil d'agitation entraîné magnétiquement, ce dernier, placé à l'intérieur du réservoir, comprend un élément magnétique à proximité du fond du réservoir avec agitateur, qui est actionné par un élément magnétique correspondant placé sur un moteur d'entraînement à l'extérieur du réservoir. L'activation du moteur d'entraînement qui possède l'élément magnétique correspondant positionné à proximité de l'élément magnétique de l'appareil d'agitation, entraîne la rotation de ce dernier à l'intérieur du réservoir avec agitateur.

Encore plus récemment, des réservoirs avec agitateurs stériles ont été mis au point pour utiliser un récipient flexible comme conteneur de mélange. Les récipients flexibles peuvent être construits dans un environnement stérile et fermés hermétiquement avant utilisation. Ces systèmes, qui utilisent un support de réservoir pour maintenir l'intégrité du conteneur flexible lorsqu'il est rempli, sont généralement jetés après utilisation pour s'affranchir du besoin de nettoyage, de manière à recréer un environnement stérile dans le récipient entre les utilisations. La capacité de contrôle de l'environnement stérile est donc grandement améliorée.

De plus, on sait que les réservoirs avec agitateur destinés à être utilisés dans des applications stériles comprennent l'appareil d'agitation dans le récipient fermé hermétiquement lors de l'expédition. Dans ces réservoirs avec agitateur, l'appareil d'agitation stérile est placé à l'intérieur du récipient stérile avant fermeture hermétique, réduisant le risque de rupture de l'environnement stérile.

Le document US-A-4670397 décrit un récipient-mélangeur destiné à recevoir un contenu biopharmaceutique. Ce document ne révèle pas un port combiné vidange/palier inférieur ni un élément tubulaire d'amenée de gaz d'aération qui s'étend à partir d'un élément étendu de distribution dans l'espace intérieur. Le problème est donc d'optimiser tant le mélange que l'aération y compris dans le cas de conteneurs de grande capacité.

Des exemples de réservoirs avec agitateur comportant un appareil d'agitation à fluide interne entraîné magnétiquement sont présentés dans les documents US-A-4209259, US-A-4993841, US-A- 5470152, JP-A-56-045752 et WO 03/02886AZ. Chacune de ses références décrit des réservoirs avec agitateur comportant un appareil d'agitation ayant des éléments magnétiques entraînés, activés par des éléments magnétiques d'entraînement coopérants voisins associés à un moyen d'entraînement.

Parmi les références citées, le document US-A-5470152 décrit un réservoir avec agitateur comportant un boîtier d'entraînement dans lequel l'élément d'entraînement est inséré. Un rotor possédant un élément magnétique est fixé au boîtier d'entraînement, l'élément magnétique comprenant des aimants orientés verticalement, de manière à être parallèle à l'axe longitudinal du boîtier d'entraînement contenant l'élément magnétique coopérant. Tel qu'illustré et décrit dans la référence, le rotor est fixé de manière amovible à la partie inférieure du boîtier d'entraînement par une attache.

Les documents US-A-4209259 et US-A-4993841 décrivent des récipients de mélange avec un appareil d'agitation entraîné magnétiquement, sous forme de rotors montés sur des montants dans les récipients. Chacune de ces références décrit l'appareil d'agitation comme étant situé à l'intérieur du récipient, dans une zone voisine d'une bride ou d'un logement qui positionne le rotor par rapport au moyen d'entraînement. Les rotors de ces références se trouvent cependant dans le récipient en une position unique sur un montant et peuvent être retirés en tirant sur une bague placée à l'extrémité terminale de l'appareil d'agitation.

De manière similaire, le dispositif du document WO 03/028869 AZ utilise un rotor reçu par un montant placé sur une partie rigide du récipient de mélange. La partie restante du récipient de mélange est désignée comme étant une partie flexible, décrite dans la référence comme un sac. Le rotor comporte un élément magnétique entraîné par un moteur d'entraînement extérieur possédant un élément d'entraînement magnétique.

Le document JP-A-56-045752 concerne un dispositif d'agitation entraîné magnétiquement possédant une plaque circulaire rotative sur des roulements à billes fixés à la partie inférieure du récipient, où l'élément magnétique de l'appareil d'agitation est associé à la partie inférieure du récipient. L'appareil d'agitation de cette référence est formé d'un alliage métallique destiné à être usé.

Aucune des références de l'art antérieur ne décrit cependant un récipient de mélange à usage unique fermé hermétiquement et stérile, comprenant un axe central fixé aux parties supérieure et inférieure du récipient et utilisant des paliers de butée ou radiaux, tels que paliers lisses, paliers à billes, ou paliers à rouleaux, pour faciliter la rotation de l'arbre, sur lequel un ou plusieurs rotor(s) est (sont) monté(s). De plus, aucune des références ne décrit un récipient de mélange à usage unique possédant un orifice de vidange incorporé dans l'élément de fixation inférieur de l'arbre pour vider le récipient, une fois le processus de mélange réalisé.

Par ailleurs, le document US-A-2006/0270036 décrit un récipient-mélangeur qui, dans une réalisation comprend:
■ un dispositif rigide extérieur de contention comprenant une paroi de fond, une paroi périphérique et une ouverture supérieure, délimitant un logement
■ un conteneur flexible disposé dans le logement, comportant :
   o une paroi ayant une partie inférieure, une partie latérale et une partie supérieure, délimitant un espace intérieur destiné à recevoir une certaine quantité du contenu,
   o des ports d'introduction dans le conteneur de composants du contenu et de vidange du contenu ménagés dans la partie supérieure de la paroi du conteneur,
■ des moyens de mélange du contenu, comportant un arbre dressé, agencé pour d'une part être entraîné à rotation par des moyens moteurs mécaniques intégralement disposés à l'extérieur du conteneur, et d'autre part entraîner à rotation un disque mélangeur à volets flexibles, situé dans l'espace intérieur, au voisinage immédiat de la partie inférieure de la paroi du conteneur, monté de façon à pouvoir monter ou descendre.
■ des moyens d'aération agencés pour délivrer au contenu une certaine quantité de gaz d'aération, comportant d'une part des moyens d'amenée de gaz d'aération ayant un élément tubulaire traversant la partie inférieure de la paroi du conteneur entre l'espace interne et l'extérieur, et d'autre part, en communication fluidique, des moyens de distribution de gaz d'aération comprenant un élément de distribution ayant en section droite transversale une forme de ∩ à branches divergentes, dont la paroi laisse passer des bulles du gaz d'aération provenant des moyens d'amenée, situé dans l'espace intérieur en étant attenant de la partie inférieure de la paroi du conteneur, et immédiatement en dessous du disque mélangeur.

Un tel récipient-mélangeur présente plusieurs inconvénients. Le disque mélangeur est situé vers la partie inférieure du conteneur ce qui limite le mélange obtenu et se révèle particulièrement inapproprié dans le cas où l'on souhaite avoir un conteneur de grande capacité, par exemple pouvant atteindre 5.000 litres. L'inconvénient est d'autant plus réel que l'élément de distribution de gaz d'aération est confiné entre la partie inférieure de la paroi du conteneur et le disque mélangeur.

Le document WO 2008/088371 décrit un récipient-mélangeur du même type général et présentant par conséquent les mêmes inconvénients.

Le document US-5206172 décrit un récipient de fermentation comprenant des moyens de distribution de gaz d'aération de forme torique. Un tel récipient correspond à un usage bien déterminé et il ne prévoit pas la présence et la mise en oeuvre de moyens de mélange.

L'invention vise à résoudre les problèmes posés par les récipients-mélangeurs connus du type comportant des moyens d'aération, et, plus particulièrement, à optimiser tant le mélange que l'aération et ce, y compris dans le cas de conteneurs de grande capacité par exemple pouvant atteindre 5.000 litres.

A cet effet, selon un premier aspect, l'invention vise un récipient-mélangeur destiné à recevoir un contenu biopharmaceutique en vue de son mélange, comprenant:
■ un conteneur flexible, comportant :
   o une paroi ayant une partie inférieure, une partie latérale et une partie supérieure, délimitant un espace intérieur apte à recevoir une certaine quantité du contenu,
   o un ou plusieurs ports d'introduction dans le conteneur du contenu ou de composants du contenu, coopérant avec un ou plusieurs orifices d'introduction ménagés dans le conteneur,
   o au moins un port de vidange du contenu coopérant avec au moins un orifice de vidange,
■ des moyens de mélange du contenu, comportant :
   o au moins un arbre dressé, apte à être entraîné à rotation par des moyens moteurs et à entraîner à rotation au moins un organe de mélange,
   o au moins un palier inférieur, adjacent à la partie inférieure de la paroi du conteneur, avec lequel coopère la partie inférieure de l'arbre,
   o au moins un organe de mélange, apte à agiter le contenu, situé dans l'espace intérieur,
■ des moyens d'aération aptes à délivrer au contenu une certaine quantité de gaz d'aération, comportant :
   o des moyens d'amenée de gaz d'aération ayant au moins un élément tubulaire s'étendant avec communication fluidique depuis l'extérieur du conteneur jusqu'aux moyens de distribution,
   o des moyens de distribution de gaz d'aération comprenant au moins un élément étendu de distribution dont la paroi laisse passer des bulles du gaz d'aération provenant des moyens d'amenée, situé dans l'espace intérieur vers la partie inférieure de la paroi du conteneur, caractérisé par le fait que :
■ il comporte au moins un port combiné vidange/palier inférieur ayant une flasque rigide :
   o pourvue d'un passage de vidange en communication fluidique d'un côté avec l'espace intérieur et de l'autre avec l'extérieur du conteneur,
   o fixée de façon rigide et étanche à la partie inférieure de la paroi du conteneur autour de l'orifice de vidange, le passage de vidange et l'ouverture de vidange étant en communication fluidique,
   o supportant du côté intérieur un palier inférieur situé dans l'espace intérieur, adjacent au passage de vidange sans empêcher la communication fluidique entre le passage de vidange et l'ouverture de vidange,
■ le au moins un élément étendu de distribution de gaz d'aération est substantiellement espacé radialement du port vidange/palier,
■ le au moins un élément tubulaire d'amenée de gaz d'aération s'étend à partir de l'élément étendu de distribution, dans l'espace intérieur, le long de la face intérieure de la partie inférieure et de la partie latérale de la paroi du conteneur et s'étend à l'extérieur du conteneur à partir de la - ou du voisinage de la - partie supérieure de la paroi du conteneur,
■ au moins un organe de mélange est substantiellement espacé de la partie inférieure de la paroi du conteneur, du palier inférieur et du au moins un élément étendu de distribution de gaz d'aération, de manière que les bulles du gaz d'aération distribuées depuis le au moins un élément étendu de distribution du gaz d'aération, soient réparties dans le contenu par une première répartition dans la zone inférieure de l'espace intérieur adjacente à la partie inférieure de la paroi du conteneur, par le au moins un élément étendu de distribution du gaz de distribution et une seconde répartition par le au moins un organe de mélange dans l'ensemble de l'espace intérieur du conteneur.

Selon une première réalisation, le au moins un arbre des moyens de mélange coopère avec un unique palier - le palier inférieur -, le moyen moteur d'entraînement à rotation de l'arbre étant situé vers la partie inférieure de la paroi du conteneur.

Selon une seconde réalisation, le au moins un arbre des moyens de mélange coopère avec deux paliers, sa partie inférieure avec le palier inférieur et sa partie supérieure avec un palier supérieur adjacent à la partie supérieure.

Selon une réalisation, le récipient-mélangeur comporte au moins un palier supérieur, ayant une flasque rigide fixée de façon rigide à la partie supérieure de la paroi du conteneur, supportant du côté intérieur un palier supérieur situé dans l'espace intérieur.

Selon une réalisation, le récipient-mélangeur comporte au moins un port combiné introduction/palier supérieur ayant une flasque rigide :
o pourvue d'un passage d'introduction du contenu ou de composants du contenu en communication fluidique d'un côté avec l'espace intérieur et de l'autre avec l'extérieur du conteneur,
o fixée de façon rigide et étanche à la partie supérieure de la paroi du conteneur autour de l'orifice d'introduction, le passage d'introduction et l'ouverture d'introduction étant en communication fluidique,
o supportant du côté intérieur le palier supérieur situé dans l'espace intérieur, adjacent au passage d'introduction sans empêcher la communication fluidique entre le passage d'introduction et l'ouverture d'introduction.

Selon les réalisations, le moyen moteur d'entraînement à rotation de l'arbre est situé vers la partie supérieure et/ou vers la partie inférieure de la paroi du conteneur.

Selon une première réalisation, le au moins un arbre des moyens de mélange est situé tout entier dans l'espace intérieur, le moyen moteur d'entraînement à rotation de l'arbre étant à fonctionnement magnétique, un disque rotatif menant à pôles magnétiques, situé à l'extérieur du conteneur, coopérant fonctionnellement avec un disque rotatif mené à pôles magnétiques, fixé sur le au moins un arbre à proximité magnétique du disque rotatif menant.

Selon une seconde réalisation, le au moins un arbre des moyens de mélange est situé pour partie dans l'espace intérieur et pour partie à l'extérieur du conteneur, le moyen moteur d'entraînement à rotation de l'arbre étant à fonctionnement mécanique, un arbre rotatif menant, situé à l'extérieur du conteneur, coopérant fonctionnellement avec la partie extérieure du au moins un arbre.

Selon une première réalisation, les moyens de mélange comportent un unique arbre dressé. Selon une seconde réalisation, les moyens de mélange comportent plusieurs arbres dressés d'axes substantiellement parallèles, aptes à entraîner à rotation chacun au moins un organe de mélange.

Selon une première réalisation, un arbre des moyens de mélange supporte et entraîne un unique organe de mélange situé en une unique localisation axiale sur l'arbre. Selon une seconde réalisation, un arbre des moyens de mélange supporte et entraîne plusieurs organes de mélange situés en une pluralité de localisations axiales sur l'arbre.

Selon une réalisation, un organe de mélange est substantiellement espacé de la partie inférieure de la paroi du conteneur, du palier inférieur et du au moins un élément étendu de distribution, d'une distance de l'ordre d'au moins le quart de l'écartement entre la partie inférieure et la partie supérieure de la paroi du conteneur.

Selon une réalisation, un organe de mélange est substantiellement espacé de la partie inférieure de la paroi du conteneur, du palier inférieur et du au moins un élément étendu de distribution, d'une distance de l'ordre d'au moins le tiers de l'écartement entre la partie inférieure et la partie supérieure de la paroi du conteneur.

Selon une réalisation, le au moins un élément tubulaire d'amenée de gaz d'aération s'étend dans l'espace intérieur, en étant substantiellement maintenu attenant ou adjacent à la face intérieure de la paroi du conteneur.

Selon les réalisations, le au moins un élément tubulaire d'amenée de gaz d'aération est au moins pour partie structurellement distinct de la paroi du conteneur et maintenue à elle par collage, soudage ou au moyen de pièces de maintien rapportées et/ou au moins pour partie structurellement partie intégrante de la paroi du conteneur.

Selon une réalisation, le au moins un élément tubulaire d'amenée de gaz d'aération traverse la paroi du conteneur par une liaison étanche.

Selon une réalisation, le au moins un élément tubulaire d'amenée de gaz d'aération traverse la paroi du conteneur dans la partie supérieure.

Selon une réalisation, le au moins un élément étendu de distribution de gaz d'aération est maintenu attenant ou adjacent à la face intérieure de la partie inférieure de la paroi du conteneur.

Selon les réalisations, le au moins un élément étendu de distribution de gaz d'aération est, au moins pour partie, structurellement distinct de la paroi du conteneur et maintenu à elle par collage, soudage ou au moyen de pièces de maintien rapportées et/ou au moins pour partie, structurellement partie intégrante de la paroi du conteneur.

Selon une réalisation, le au moins un élément étendu de distribution de gaz d'aération ne traverse pas la paroi du conteneur.

Selon une réalisation, le au moins un élément étendu de distribution de gaz d'aération comporte une paroi pourvue d'une pluralité de trous répartis aptes au passage des bulles du gaz d'aération provenant des moyens d'amenée.

Selon une réalisation, la pluralité de trous aptes au passage des bulles du gaz d'aération provenant des moyens d'amenée est orientée avec différents axes d'inclinaison sur la verticale.

Selon les réalisations, les trous de la pluralité de trous sont soit de même taille soit de tailles différentes.

Selon une réalisation, le au moins un élément étendu de distribution de gaz d'aération a, en section droite transversale, une forme circulaire, ou pseudo-circulaire, ou elliptique ou pseudo-elliptique.

Selon une première réalisation, le au moins un élément étendu de distribution de gaz d'aération comprend au moins un anneau complet fermé sur lui-même, en communication circulaire continue ou non. Selon une seconde réalisation, le au moins un élément étendu de distribution de gaz d'aération comprend au moins un anneau incomplet ouvert par rapport à lui-même. Dans ce cas, et selon une réalisation, l'anneau incomplet a une ouverture d'angle comprise entre environ 180° et 270°.

Selon une réalisation, le au moins un élément étendu de distribution de gaz d'aération comprend au moins un anneau et au moins un élément transversal en communication fluidique.

Selon une réalisation, le au moins un anneau du au moins un élément étendu de distribution de gaz d'aération est sensiblement centré sur le port combiné vidange/palier.

Selon une première réalisation, les moyens d'aération comportent un unique ensemble de moyens d'amenée de gaz d'aération et de moyens de distribution de gaz d'aération. Selon une seconde réalisation, les moyens d'aération comportent plusieurs ensembles distincts de moyens d'amenée d'un ou de plusieurs gaz d'aération et de moyens de distribution du ou des gaz d'aération.

Selon les réalisations, un ensemble de moyens d'aération comporte un seul élément tubulaire d'amenée de gaz d'aération communiquant avec un seul élément étendu de distribution de gaz d'aération, ou un seul élément tubulaire d'amenée de gaz d'aération communiquant avec plusieurs éléments étendus de distribution de gaz d'aération, ou plusieurs éléments tubulaires d'amenée de gaz d'aération communiquant avec un seul élément étendu de distribution de gaz d'aération, ou plusieurs éléments tubulaires d'amenée de gaz d'aération communiquant avec plusieurs éléments étendus de distribution de gaz d'aération.

Selon une réalisation, dans le cas où le récipient-mélangeur comporte plusieurs éléments étendus de distribution de gaz d'aération distincts, au moins certains des plusieurs éléments étendus de distribution de gaz d'aération sont situés en une pluralité de localisations radiales dans l'espace intérieur vers la partie inférieure du conteneur.

Selon une réalisation, les plusieurs éléments étendus de distribution de gaz d'aération distincts sont substantiellement espacés radialement du port vidange/palier jusqu'au voisinage de la partie latérale de la paroi du conteneur.

Selon une réalisation, un élément étendu de distribution de gaz d'aération est substantiellement espacé radialement du port vidange/palier, d'une distance de l'ordre d'au moins le cinquième du diamètre de la partie inférieure de la paroi du conteneur.

Selon une réalisation, ne saille sous la partie inférieure de la paroi du conteneur que la vidange et, le cas échéant, le moyen moteur d'entraînement des moyens de mélange lorsqu'il est prévu en partie inférieure.

Selon une réalisation, le récipient-mélangeur comporte également un ou plusieurs ports d'évacuation de gaz coopérant avec au moins un orifice d'évacuation ménagé dans la partie supérieure de la paroi du conteneur, pourvu d'une valve anti-retour, empêchant l'introduction dans le conteneur de fluides ou de contaminants non souhaités ou indésirables.

Selon une réalisation, le récipient-mélangeur comporte également un ou plusieurs ports d'introduction, de vidange, de montage.

Selon une réalisation, le conteneur est de grande capacité, pouvant aller jusqu'à 5.000 litres.

Selon une réalisation, le récipient-mélangeur comporte également un dispositif rigide extérieur de contention du conteneur empli de son contenu, comprenant une paroi de fond, une paroi périphérique et une ouverture supérieure, délimitant un logement principal dans lequel est placé de façon amovible le conteneur flexible dont la partie inférieure repose sur la paroi de fond et dont la partie latérale vient s'appliquer, lorsque le conteneur est empli de son contenu, contre la paroi périphérique.

Dans ce cas et selon une réalisation, le dispositif rigide extérieur de contention comporte également un logement secondaire en dessous de la paroi de fond de logement et de protection de la vidange et, le cas échéant, du moyen moteur d'entraînement des moyens de mélange lorsqu'il est prévu en partie inférieure.

Selon une réalisation, le dispositif rigide extérieur de contention comporte également des moyens de chauffage et le conteneur flexible est en un matériau présentant une certaine conductivité thermique, de manière que la mise en oeuvre des moyens de chauffage permette le chauffage du contenu ; et, le cas échéant, des moyens de contrôle de la température du conteneur et des moyens de commande des moyens de chauffage.

Selon une caractéristique, le conteneur peut se trouver dans trois états extrêmes: un état désassemblé du dispositif rigide extérieur de contention dans lequel le conteneur peut être disposé de façon aplatie sur lui-même, un état assemblé au dispositif rigide extérieur de contention dans lequel le conteneur, vide de contenu, est disposé dans le logement principal du dispositif de contention en reposant sur la paroi de fond, et un état assemblé au dispositif rigide extérieur de contention dans lequel le conteneur, empli de son contenu, est disposé dans le logement principal du dispositif de contention en reposant sur la paroi de fond et en étant appliqué contre la paroi périphérique.

Selon un mode particulier de mise en oeuvre du récipient-mélangeur, on y réalise une bioréaction, le récipient-mélangeur étant un bioréacteur.

Selon un second aspect, l'invention vise un procédé de mise en oeuvre d'un récipient-mélangeur tel qu'il vient d'être décrit, dans lequel :
■ on dispose d'un tel récipient-mélangeur dont le port de vidange est obturé,
■ on dispose du contenu ou des composants du contenu destinés à être reçus dans le conteneur du récipient-mélangeur puis mélangé,
■ on introduit dans le conteneur le contenu ou des composants du contenu,
■ on met en oeuvre les moyens de mélange pour agiter le contenu du conteneur,
■ on met en oeuvre les moyens d'aération pour délivrer au contenu une certaine quantité de gaz d'aération, , l'aération et le mélange étant réalisés au moins pour partie simultanément, et
■ on distribue les bulles du gaz d'aération depuis le au moins un élément étendu de distribution du gaz d'aération et on les répartit dans le contenu par une première répartition dans la zone inférieure de l'espace intérieur adjacente à la partie inférieure de la paroi du conteneur, par le au moins un élément étendu de distribution du gaz d'aération et une seconde répartition par le au moins un organe de mélange dans l'ensemble de l'espace intérieur du conteneur.

Selon une réalisation, on introduit d'abord dans le conteneur un composant ou une partie des composants du contenu, on met en oeuvre les moyens de mélange et les moyens d'aération et on introduit pour délivrer au contenu une certaine quantité de gaz d'aération, et on introduit dans le conteneur le ou les composants restant du contenu.

Selon une réalisation :
■ on part d'un récipient-mélangeur dont le conteneur est désassemblé d'un dispositif rigide extérieur de contention, vide de contenu, et disposé de façon aplatie sur lui-même,
■ on assemble le conteneur au dispositif rigide extérieur de contention, en le disposant dans le logement principal de celui-ci, en reposant sur sa paroi de fond,
■ on introduit alors dans le conteneur le contenu ou des composants du contenu.

Selon une réalisation, l'on dispose sous la partie inférieure de la paroi du conteneur la vidange le moyen moteur d'entraînement des moyens de mélange lorsqu'il est prévu en partie inférieure.

On décrit maintenant plusieurs modes de réalisation de l'invention à l'aide des dessins, dans lesquels :
- La figure 1 est une vue en perspective d'une réalisation possible d'un récipient-mélangeur, dont le dispositif rigide extérieur de contention n'est pas représenté.
- La figure 2 est une vue analogue à la figure 1 d'une autre réalisation possible d'un récipient-mélangeur.
- La figure 3 est une vue analogue aux précédentes d'une autre réalisation possible d'un récipient-mélangeur.
- La figure 4 est une vue analogue aux précédentes d'une autre réalisation possible d'un récipient-mélangeur.
- La figure 5 est une vue en perspective partielle des moyens d'aération d'un récipient-mélangeur.
- La figure 6 est une vue en perspective partielle des moyens d'aération d'un récipient-mélangeur avec des moyens d'amenée en forme de manchon.
- Les figures 7A, 7B et 7C sont trois vues en perspective de trois modes de réalisation d'un port combiné vidange/palier destiné à faire partie du récipient-mélangeur.
- La figure 7D est une vue en coupe, partielle, montrant un port combiné vidange/palier, en situation dans un conteneur représenté de façon partielle, et les moyens d'aération.
- La figure 8 est une vue en perspective d'un ensemble comportant un port combiné vidange/palier et un port combiné introduction/palier.
- La figure 9A est une vue en coupe schématique de la flasque supérieure et des moyens magnétiques d'entraînement de l'arbre des moyens de mélange.
- La figure 9B est une vue en coupe agrandie d'une réalisation du raccordement entre la flasque supérieure et des moyens magnétiques d'entraînement de l'arbre.
- La figure 10 est une vue en coupe axiale partielle d'un autre mode de réalisation d'un récipient-mélangeur.
- La figure 11A est une vue en coupe partielle de la zone de la flasque supérieure de la réalisation illustrée sur la figure 10.
- La figure 11 B est une coupe partielle de la zone de la flasque inférieure de la réalisation illustrée sur la figure 10.

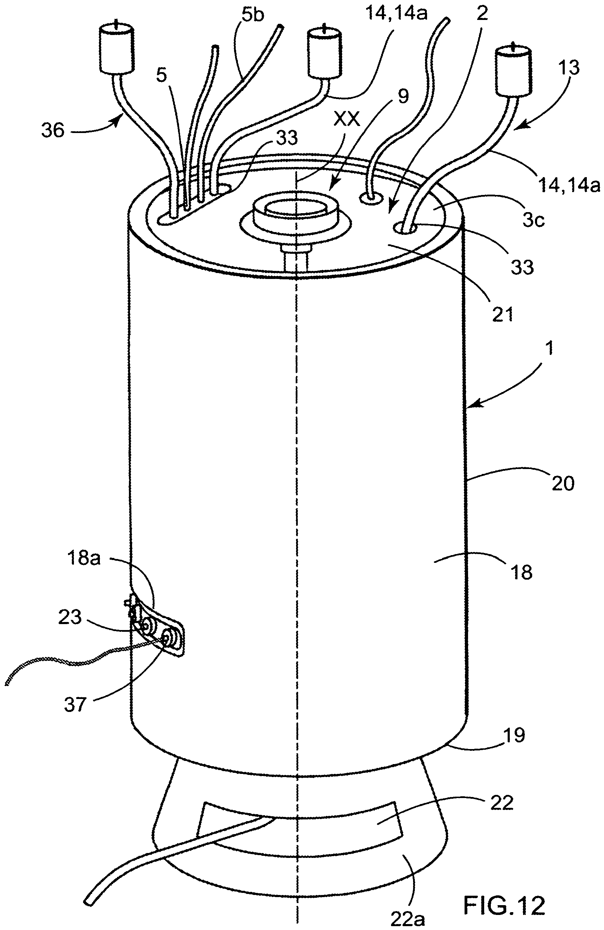

- La figure 12 est une vue en perspective extérieure du récipient-mélangeur montrant le dispositif rigide extérieur de contention.

Un récipient-mélangeur 1 selon l'invention est destiné à recevoir un contenu biopharmaceutique C en vue de son mélange ou, le cas échéant en vue d'une bioréaction, le récipient-mélangeur 1 étant alors un bioréacteur.

Le contenu C comprend une ou au moins une phase liquide. Le cas échéant, le contenu C est réalisé à partir de plusieurs composants C₁, C₂... dont au moins un est en phase liquide et dont un ou plusieurs peut être en phase solide, tel que de la poudre. Le cas échéant, dans le cas d'un bioréacteur, le contenu C comprend également des cellules, des micro-organismes...

Le récipient-mélangeur 1 présente un axe principal XX, vertical.

Le récipient-mélangeur 1 comporte en premier lieu un conteneur flexible 2. Ce conteneur flexible 2 est formé par une paroi 3, en un ou plusieurs tronçons solidarisés les uns avec les autres, ayant une partie inférieure 3a, une partie latérale 3b et une partie supérieure 3c, délimitant un espace intérieur 4, apte à recevoir une certaine quantité du contenu C.

Selon une réalisation, le conteneur flexible 2 est à usage unique.

Le conteneur flexible 2 peut avoir une capacité allant jusqu'à 5.000 litres, en fonction des besoins et des applications.

Les mots « vertical », « horizontal », « supérieur », « inférieur » se réfèrent à la situation dans laquelle le récipient-mélangeur 1 est dans une position apte à son fonctionnement. Il est entendu toutefois que le récipient-mélangeur 1 peut occuper d'autres positions ou avoir d'autres états, par exemple parce qu'il n'est pas en fonctionnement. Le mot « vertical » ne doit pas être compris dans un sens étroit, mais dans le sens signifiant du plus haut au plus bas et inversement.

D'autre part, les mots « intérieur » et « extérieur » se réfèrent, respectivement à ce qui se trouve dans et hors du conteneur 2.

Enfin, les mots « axial » d'une part, « radial » et « transversal » d'autre part, se réfèrent à ce qui s'étend dans ou parallèlement ou sensiblement parallèlement à l'axe XX d'une part, et perpendiculairement ou orthogonalement ou sensiblement perpendiculairement ou orthogonalement à l'axe XX d'autre part.

Le récipient-mélangeur 1 comporte un ou plusieurs ports 5 traversant, d'introduction dans le conteneur 2 du contenu C ou de composants C₁, C₂... du contenu C, coopérant avec un ou plusieurs orifices d'introduction 5a ménagés dans le conteneur 2.

Le récipient-mélangeur 1 comporte également au moins un port 6 traversant de vidange du contenu C du conteneur 2, coopérant avec au moins un orifice 6a de vidange ménagé dans le conteneur 2. Naturellement, le port de vidange 6 est apte à être obturé à chaque fois que nécessaire et au contraire ouvert pour la vidange.

On entend dans ce document par « port », un moyen de connexion ou de liaison physique. Un tel port est traversant lorsqu'il s'agit d'assurer une fonction de mise en communication entre l'intérieur et l'extérieur du conteneur 2, par exemple pour l'introduction ou la vidange de ce qui doit être placé ou est placé dans le conteneur 2. Un tel port peut également être non traversant lorsqu'il s'agit d'assurer une fonction de maintien d'un organe du récipient-mélangeur.

Aux ports 5 peuvent être associé, en communication fluidique et avec une connexion étanche et le cas échéant amovible, des conduits, poches, réservoirs 5b, le cas échéant souples. De même, aux ports 6 peuvent être associé, en communication fluidique et avec une connexion étanche et le cas échéant amovible, des conduits, poches, réservoirs 6b, le cas échéant souples. Ces conduits, poches, réservoirs 5b et 6b sont situés et s'étendent à l'extérieur du récipient-mélangeur 1 et reliés de façon appropriée à des amenées et évacuations, respectivement. Ces conduits, poches, réservoirs 5b, 6b, sont adaptés - notamment en ce qui concerne leur taille - à la nature de ce qu'ils contiennent ou assurent le passage. La connexion étanche et le cas échéant amovible est assurée par tout dispositif approprié, comme il est connu dans le domaine de l'invention.

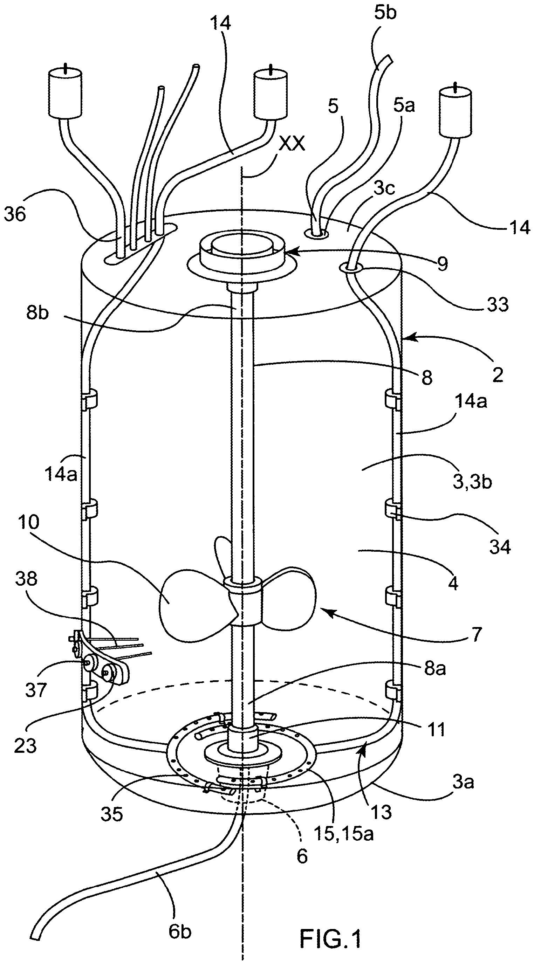

Dans la réalisation représentée sur la figure 1, les ports 5 d'introduction sont placés en position supérieure du récipient-mélangeur 1 et les orifices d'introduction 5a sont ménagés dans la partie supérieure 3c de la paroi 3, tandis que le port 6 de vidange est placé en position la plus basse du récipient-mélangeur 1 et l'orifice 6a de vidange ménagé dans la partie inférieure 3a du conteneur 2, dans sa zone la plus basse. Le cas échéant, un (ou plusieurs) port 5 d'introduction est placé en position inférieure du récipient-mélangeur 1 et l'orifice 5a d'introduction correspondant ménagé dans la partie inférieure 3a du conteneur 2 ou dans la zone inférieure de la partie latérale 3b.

Le récipient-mélangeur 1 comporte également des moyens 7 de mélange du contenu du conteneur 2. On entend par là le mélange de ce qui se trouve dans l'espace intérieur 4 du conteneur 2, qu'il s'agisse du contenu C, ou d'une partie de ses composants, et/ou d'une partie seulement de la quantité totale qui doit y être placée.

Les moyens 7 de mélange comprennent, en premier lieu, au moins un arbre 8, dressé, apte à être entraîné à rotation par des moyens moteurs 9 et à entraîner à rotation au moins un organe de mélange 10.

Les moyens 7 de mélange comprennent, en deuxième lieu, au moins un palier inférieur 11, adjacent à la partie inférieure 3a de la paroi 3, avec lequel coopère la partie inférieure 8a de l'arbre 8.

Les moyens 7 de mélange comprennent, en troisième lieu, l'au moins un organe de mélange 10, apte à agiter le contenu, situé dans l'espace intérieur 4.

Le récipient-mélangeur 1 comporte également des moyens d'aération 13 aptes à délivrer au contenu une certaine quantité de gaz d'aération. On entend par là l'aération de ce qui se trouve dans l'espace intérieur 4 du conteneur 2, qu'il s'agisse du contenu C, ou d'une partie de ses composants, et/ou d'une partie seulement de la quantité totale qui doit y être placée.

Les moyens 13 d'aération comprennent, en premier lieu, des moyens 14 d'amenée de gaz d'aération ayant au moins un élément tubulaire 14a s'étendant avec communication fluidique depuis l'extérieur du conteneur 2 jusqu'à des moyens de distribution 15.

Les moyens 13 d'aération comprennent, en second lieu, les moyens 15 de distribution de gaz d'aération comprenant au moins un élément étendu 15a de distribution dont la paroi laisse passer des bulles du gaz d'aération provenant des moyens d'amenée 14. Cet élément étendu 15a de distribution de gaz d'aération est situé dans l'espace intérieur 4, vers la partie inférieure 3a de la paroi 3 du conteneur 2.

Le récipient-mélangeur 1 comporte également au moins un port combiné vidange/palier inférieur 6+11 1 ayant une flasque rigide 16.

On entend ici par « flasque », une pièce rigide en forme générale de paroi pleine, au moins sensiblement plate, placée à plat, et destinée au maintien.

La flasque 16 est, en premier lieu, pourvue d'un passage 17 de vidange. Ce passage 17 est en communication fluidique d'un côté avec l'espace intérieur 4 du conteneur 2 et, de l'autre côté, avec l'extérieur du conteneur 2. C'est ainsi que la vidange du contenu du conteneur 2 à l'extérieur du récipient-mélangeur est possible.

La flasque 16 est, en deuxième lieu, fixée de façon rigide et étanche à la partie inférieure 3a de la paroi 3 du conteneur 2, autour de l'orifice de vidange 6a. Le passage de vidange 17 et l'orifice de vidange 6a sont en communication fluidique.

La flasque 16, en troisième lieu, supporte, du côté intérieur le palier inférieur 11, lequel est situé dans l'espace intérieur 4 et est adjacent au passage de vidange 17, sans empêcher la communication fluidique entre le passage de vidange et l'ouverture de vidange.

Par ailleurs, le au moins un élément étendu 15a de distribution de gaz d'aération est substantiellement espacé radialement du port vidange/palier 6+11. On entend par là que l'élément étendu 15a n'est pas incorporé dans ou à proximité immédiate du port vidange/palier 6+11.

Le au moins un élément tubulaire 14a d'amenée de gaz d'aération s'étend à partir de l'élément étendu 15a de distribution, dans l'espace intérieur 4, le long de la face intérieure de la partie inférieure 3a et de la partie latérale 3b de la paroi 3 du conteneur 2. Le au moins un élément tubulaire 14a d'amenée de gaz d'aération s'étend à l'extérieur du conteneur 2 à partir de la - ou du voisinage de la - partie supérieure 3c de la paroi 3 du conteneur 2.

Au moins un organe de mélange 10 est substantiellement espacé de la partie inférieure 3a de la paroi 3 du conteneur 2, du palier inférieur 11 et du au moins un élément étendu 15a de distribution de gaz d'aération. On entend par là que l'organe de mélange 10 n'est pas incorporé dans ou à proximité immédiate de la paroi 3 du conteneur 2, du palier inférieur 11 et du au moins un élément étendu 15a de distribution de gaz d'aération.

Les dispositions constructives qui précèdent sont telles que les bulles du gaz d'aération distribuées depuis le au moins un élément étendu 15a de distribution du gaz d'aération, sont réparties dans le contenu de l'espace intérieur 4 par, d'une part, une première répartition dans la zone inférieure de l'espace intérieur 4 adjacente à la partie inférieure 3a de la paroi 3 du conteneur 2, par le au moins un élément étendu 15a de distribution du gaz de distribution et, d'autre part, une seconde répartition par le au moins un organe de mélange 10 dans l'ensemble de l'espace intérieur 4 du conteneur 2.

Le récipient-mélangeur 1 comporte également, en raison de la nature flexible du conteneur 2, un dispositif rigide - éventuellement semi-rigide - extérieur de contention 18 du conteneur 2 empli de son contenu pendant le remplissage, le mélange et la vidange.

Le dispositif rigide extérieur de contention 18 comprend une paroi de fond 19 et une paroi périphérique 20, ménageant une ouverture supérieure d'accès 21 et délimitant un logement principal dans lequel est placé de façon amovible le conteneur flexible 2.

Le dispositif rigide extérieur de contention 18 est généralement de géométrie, forme et et/ou dimension identiques au conteneur flexible 2, afin de réduire les sollicitations sur les soudures ou les changements de direction dans la matière du conteneur flexible 2.

Le dispositif rigide extérieur de contention 18 comporte l'ouverture d'accès 21 afin de permettre la mise en place et l'enlèvement du conteneur flexible 2.

Le cas échéant, le dispositif rigide extérieur de contention 18 comporte d'autres ouvertures pour introduire le contenu C ou les composants C₁, C₂... du contenu C et vidanger le contenu C, ou pour accéder aux différents éléments du récipient-mélangeur 1 qui doivent être accessibles pour l'utilisation, y compris, le moyen 9 moteur, les conduits, poches, réservoirs 5b et 6b, les autres organes.

La partie inférieure 3a de la paroi 3 du conteneur 2 repose sur la paroi de fond 19, tandis que la partie latérale 3b de la paroi 3 du conteneur 2 vient s'appliquer, lorsque le conteneur 2 est empli de son contenu, contre la paroi périphérique 20.

Le cas échéant, le dispositif rigide extérieur de contention 18 comporte également, ou forme, un logement ou espace secondaire 22 situé en dessous de la paroi de fond 19. Ce logement ou espace secondaire 22 permet le logement et la protection des moyens du récipient-mélangeur 1 se trouvant en dessous du conteneur 2. Il s'agit par exemple du conduit, de la poche ou du réservoir 6b associé à la vidange et, le cas échéant, du moyen moteur 9 d'entraînement des moyens 7 de mélange lorsque ce moyen moteur 9 d'entraînement est prévu en partie inférieure. Le cas échant, le logement ou espace secondaire 22 peut être ménagé à l'intérieur d'un piètement 22a, tel que tant la partie inférieure 3a de la paroi 3 du conteneur 2 que la paroi de fond 19 du dispositif rigide extérieur de contention 18 soit écarté du sol ou de la surface d'appui recevant le récipient-mélangeur 1, ce dernier étant maintenu en position verticale, tout en autorisant l'accès à l'orifice de vidange 6a et le cas échéant au moyen 9 moteur.

Le cas échéant, le dispositif rigide extérieur de contention 18 comporte également des moyens de chauffage destinés au chauffage du contenu du conteneur 2. Dans ce cas, le conteneur flexible est réalisé en un matériau présentant une certaine conductivité thermique, de manière que la mise en oeuvre des moyens de chauffage en question permette le chauffage du contenu. Dans ce cas, et le cas échéant, il est également prévu des moyens de contrôle de la température du contenu dans le conteneur 2 et des moyens de commande des moyens de chauffage. De tels moyens de contrôle de la température sont portés par un ou des ports 23 prévus à cet effet.

Le cas échéant, le dispositif rigide extérieur de contention 18 comporte également des portes, des fenêtres, etc. 18a

Selon une réalisation, la paroi de fond 19 a une forme de calotte arrondie, par exemple hémisphérique ou pseudo hémisphérique, la partie inférieure 3a de la paroi 3 du conteneur 2 ayant une même forme. Cette disposition constructive, combinée aux dispositions constructives précédemment exposées, contribue à l'efficacité du mélange et de l'aération.

Le conteneur flexible 2 peut se trouver dans trois états extrêmes :
- Un état désassemblé, dans lequel le conteneur 2 est désassemblé du dispositif rigide extérieur de contention 18. Dans cet état, le conteneur 2, qui est flexible dans son ensemble, peut - alors qu'il est vide du contenu C - être disposé de façon aplatie sur lui-même. Cet état est tout particulièrement utile pour le stockage ou le transport.
- Un état assemblé vide, dans lequel le conteneur 2 est assemblé au dispositif rigide extérieur de contention 18, comme il a été décrit plus haut. le conteneur 2 étant vide du contenu C. Dans cet état, le conteneur 2 est disposé dans le logement principal du dispositif de contention 18 en reposant sur la paroi de fond 19.
- et, enfin, un état assemblé empli, dans lequel le conteneur est assemblé au dispositif rigide extérieur de contention 18, comme il a été décrit plus haut, le conteneur 2 étant empli de contenu C.

Dans cet état, le conteneur 2 est disposé dans le logement principal du dispositif de contention 18 en reposant sur la paroi de fond 19 et en étant appliqué contre la paroi périphérique 20.

Pour le procédé de mise en oeuvre d'un récipient-mélangeur 1 tel qu'il vient d'être décrit, on dispose d'un tel récipient-mélangeur 1 dont le port de vidange 6 est obturé et on dispose du contenu C ou des composants C₁, C₂... du contenu C.

Ce contenu C ou ces composants C₁, C₂... du contenu C sont destinés à être reçus dans le conteneur du récipient-mélangeur 1, puis mélangé, moyennant une aération.

Selon le procédé, tout d'abord, on introduit dans le conteneur 2, le contenu C ou les composants C₁, C₂... du contenu C.

Puis, on met en oeuvre les moyens 7 de mélange pour agiter le contenu du conteneur 2 se trouvant dans l'espace intérieur 4.

Par ailleurs, on met en oeuvre les moyens 13 d'aération pour délivrer au contenu du conteneur 2 se trouvant dans l'espace intérieur 4, une certaine quantité de gaz d'aération.

L'agitation et l'aération sont réalisées au moins partiellement simultanément, le cas échéant, totalement simultanément.

Par suite des dispositions constructives du récipient-mélangeur 1 et du procédé mis en oeuvre, on distribue les bulles du gaz d'aération depuis le au moins un élément étendu 15a de distribution et on les répartit dans le contenu C se trouvant dans l'espace intérieur 4, par, d'une part, une première répartition dans la zone inférieure de l'espace intérieur 4 adjacente à la partie inférieure 3a de la paroi 3 du conteneur 2, par le au moins un élément étendu 15a de distribution du gaz de distribution et, d'autre part, une seconde répartition par le au moins un organe de mélange 10 dans l'ensemble de l'espace intérieur 4 du conteneur 2.

Le procédé peut faire l'objet de plusieurs modes d'exécution. Ainsi, on peut introduire, d'abord dans le conteneur 2 un composant composants C₁ du contenu C ou une partie des composants C₁, C₂... du contenu C, puis on met en oeuvre les moyens 7 de mélange et les moyens 13 d'aération de manière à délivrer au contenu une certaine quantité de gaz d'aération, puis on introduit dans le conteneur le ou les composants C₁, C₂... restant du contenu C.

Le récipient-mélangeur 1 comportant d'une part le conteneur flexible 2 et d'autre part le dispositif rigide extérieur de contention 18, on peut procéder ainsi.

On part d'un récipient-mélangeur 1 dont le conteneur 2 est désassemblé du dispositif rigide extérieur de contention 18, ainsi que vide de contenu et disposé de façon plus ou moins aplatie sur lui-même. Puis, on assemble le conteneur 2 au dispositif rigide extérieur de contention 18, en le disposant dans le logement principal de celui-ci, en reposant sur sa paroi de fond 19.

Enfin, on introduit alors dans le conteneur 2 le contenu C ou des composants C₁, C₂... du contenu C.

Le reste du procédé est comme décrit plus haut.

Dans le cas où, comme décrit plus haut, le dispositif rigide extérieur de contention 18 comporte également, ou forme, un logement ou espace secondaire 22 situé en dessous de la paroi de fond 19, le procédé comporte également une étape préliminaire dans laquelle on dispose sous la paroi de fond 19 les moyens du récipient-mélangeur 1 qui doivent s'y trouver, tels que le conduit, la poche ou le réservoir 6b associé à la vidange et, le cas échéant, le moyen moteur 9 d'entraînement des moyens 7 de mélange lorsque ce moyen moteur 9 d'entraînement est prévu en partie inférieure.

Le récipient-mélangeur peut faire l'objet de plusieurs réalisations en fonction des différentes variantes d'exécution des moyens 7 de mélange, du port combiné vidange/palier 6+11 et des moyens 13 d'aération, les différentes variantes de ces moyens 7 et 13 pouvant, de surcroît, être le plus souvent combinées entre elles.

On décrit maintenant plus spécialement différentes variantes d'exécution des moyens 7 de mélange.

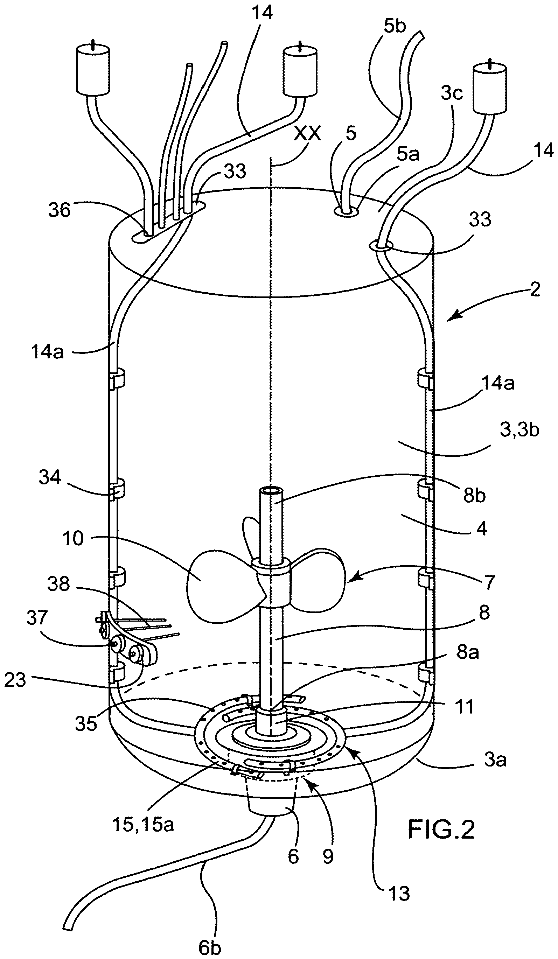

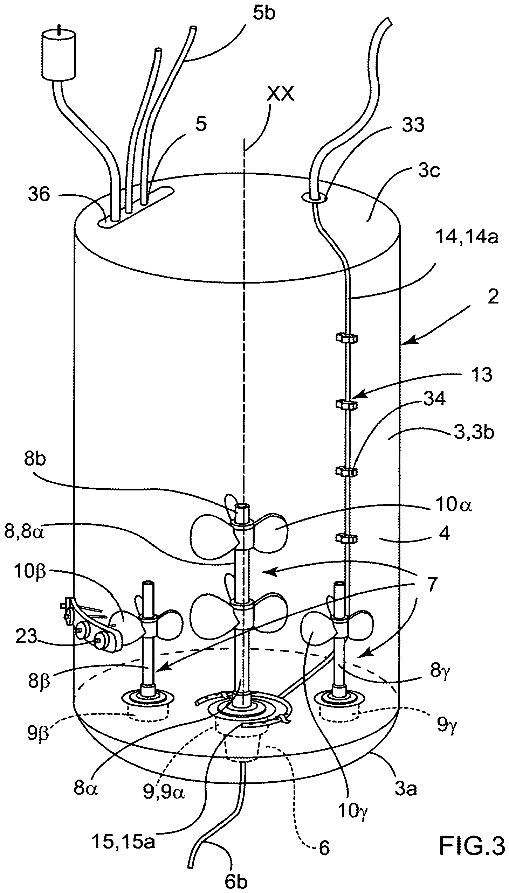

Selon une première variante d'exécution possible (figures 2 et 3), le au moins un arbre 8 des moyens 7 de mélange coopère avec un unique palier, à savoir le palier inférieur 11. Dans ce cas, l'arbre 8 comporte une extrémité libre supérieure 8b qui est écartée de la partie supérieure 3c de la paroi 3 du conteneur 2, par exemple située environ à mi-hauteur du conteneur 2. Avec une telle variante, le moyen moteur 9 d'entraînement à rotation de l'arbre 8 est situé vers la partie inférieure 3a de la paroi 3 du conteneur 2.

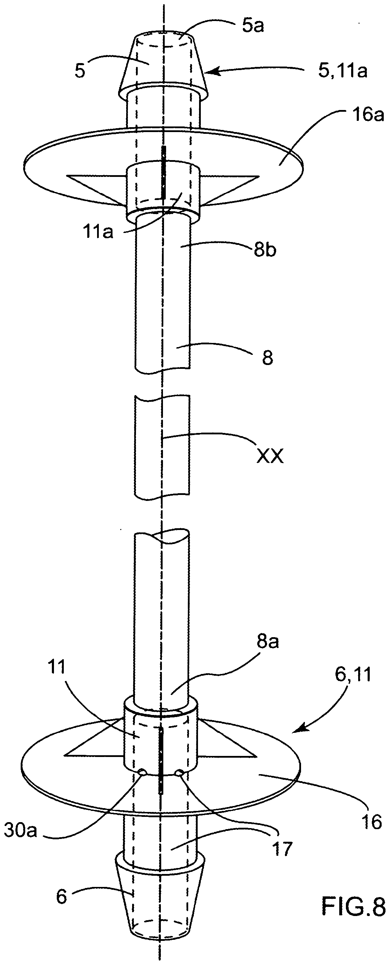

Selon une seconde variante d'exécution possible (figures 1 et 8), le au moins un arbre 8 des moyens 7 de mélange coopère avec deux paliers. Sa partie inférieure 8a coopère avec le palier inférieur 11, tandis que sa partie extrême supérieure 8b coopère avec un palier supérieur 11a, adjacent à la partie supérieure 3c.

Dans cette seconde variante, le palier supérieur 11a a, préférentiellement, une flasque 16a, rigide fixée de façon rigide à la partie supérieure 3c de la paroi 3 du conteneur 2. Cette flasque 16a supporte du côté intérieur le palier supérieur 11a qui est situé dans l'espace intérieur 4.

Selon un mode particulier de réalisation de la seconde variante (figure 8), le récipient-mélangeur 1 comporte également au moins un port combiné introduction/palier supérieur 5+11a ayant la flasque rigide 16a dont il a été question, laquelle présente une structure analogue à celle de la flasque 16 du port combiné vidange/palier inférieur 6+11.

Dans ce cas, cette flasque 16a est, en premier lieu, pourvue d'un passage d'introduction du contenu C ou de composants C₁, C₂... du contenu C, ce passage étant en communication fluidique d'un côté avec l'espace intérieur 4 et de l'autre avec l'extérieur du conteneur 2.

Cette flasque 16a est, en deuxième lieu, fixée de façon rigide et étanche à la partie supérieure 3c de la paroi 3 du conteneur 2 autour de l'orifice d'introduction 5a, le passage d'introduction et l'orifice d'introduction 5a étant en communication fluidique.

Cette flasque 16a, en troisième lieu, supporte du côté intérieur le palier supérieur 11a situé dans l'espace intérieur 4 en étant adjacent au passage d'introduction, sans empêcher la communication fluidique entre le passage d'introduction et l'orifice d'introduction 5a.

Selon d'autres variantes d'exécution possibles, le moyen moteur 9 d'entraînement à rotation de l'arbre 8 est situé vers la partie inférieure 3a (figure 2) ou vers la partie supérieure 3c (figure 1) de la paroi 3 du conteneur 2. Le cas échéant, il est prévu un moyen moteur 9 vers la partie inférieure 3a et vers la partie supérieure 3c. Si l'arbre 8 n'atteint pas la partie supérieure 3c, le moyen moteur 9 est placé vers la partie inférieure 3a. Si l'arbre 8 atteint la partie supérieure 3c, le moyen moteur 9 est placé, selon les cas, vers la partie inférieure 3a ou vers la partie supérieure 3c ou vers les deux.

Selon une autre première variante d'exécution possible (figures 1, 2, 3, 9A, 9B, 10, 11a et 11B), le au moins un arbre 8 des moyens 7 de mélange est situé tout entier dans l'espace intérieur 4. Dans ce cas, le moyen moteur 9 d'entraînement à rotation de l'arbre 8 - ici vers la partie supérieure 3c de la paroi 3 du conteneur 2 - est à fonctionnement magnétique. A cet effet, il est prévu un disque rotatif menant 9a à pôles magnétiques 9b (aimants), situé à l'extérieur du conteneur 2, coopérant fonctionnellement avec un disque rotatif mené 24 à pôles magnétiques (24a), fixé sur le au moins un arbre 8 à proximité magnétique du disque rotatif menant 9a.

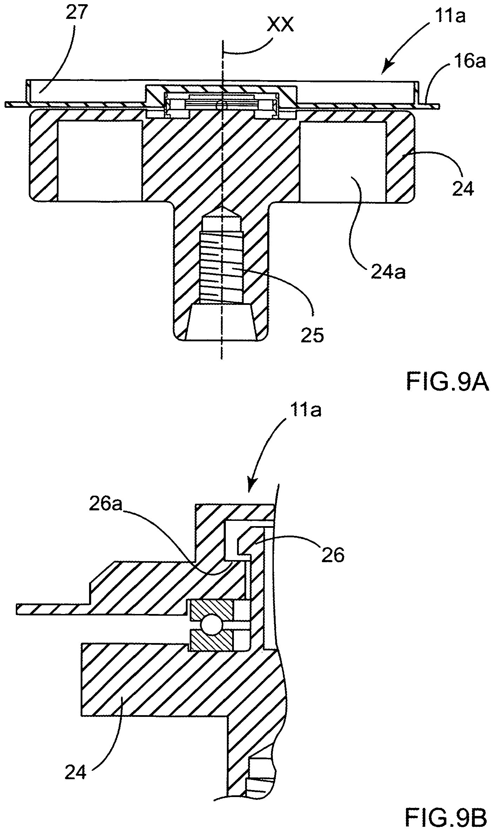

On se réfère maintenant plus spécialement aux figures 9A et 9B.

L'extrémité supérieure 8b de l'arbre 8 incorpore un disque magnétique 24 incluant une pluralité d'aimants 24a, qui est intégré par tout moyen de fixation ou de construction. Le disque magnétique 24 est ensuite positionné à proximité de la flasque supérieure 16a. Le disque magnétique 24 est raccordé à la flasque supérieure 16a, de manière à permettre au moyen moteur 9 magnétique (non représenté) d'agir sur les aimants 24a du disque magnétique 24, dans la largeur de la flasque supérieure 16a.

Selon la figure 9A, le disque magnétique 24 est fixé sur l'arbre 8 par vissage d'une extrémité filetée de l'arbre 8 dans une ouverture filetée 25 à l'intérieur du disque magnétique 24. D'autres moyens, tels qu'éléments clavetés, adhésifs, attaches, fixations rapides, goupilles, vis, verrous, soudage, et similaires, ainsi que la formation du disque magnétique 24 sur l'arbre 8 lors de sa fabrication, peuvent être utilisés pour fixer le disque magnétique 24 à l'arbre 8, sans limitation.

Pour maintenir le disque magnétique 24 en relation correcte avec la flasque supérieure 16a, il est prévu un crochet ou un cliquet 26 sur le disque magnétique 24, qui s'engage sur une lèvre 26a de la flasque supérieure 16a. Les moyens particuliers de fixation du disque magnétique 24 et de la flasque supérieure 16a consistant en l'utilisation du cliquet 26 associé à la flasque supérieure 16a et de la lèvre 26a associée au disque magnétique 24 ne sont pas exclusifs d'autres, des alternatives telles que fixations rapides, profilés et similaires étant possibles, dès lors que le disque magnétique 24 peut tourner relativement librement par rapport à la flasque supérieure 16a.

De plus, pour s'assurer que le moyen moteur 9, magnétique, (non représenté) conserve un alignement correct avec les aimants 24a du disque magnétique 24, la flasque supérieure 16a inclut, dans la réalisation représentée, un accouplement d'entraînement 27 qui s'étend vers le haut, à partir de la face extérieure de la flasque supérieure 16a.

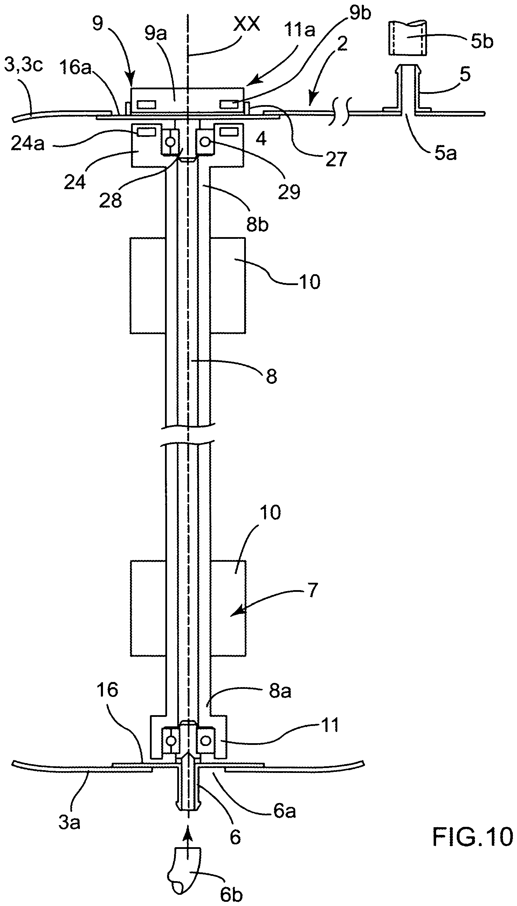

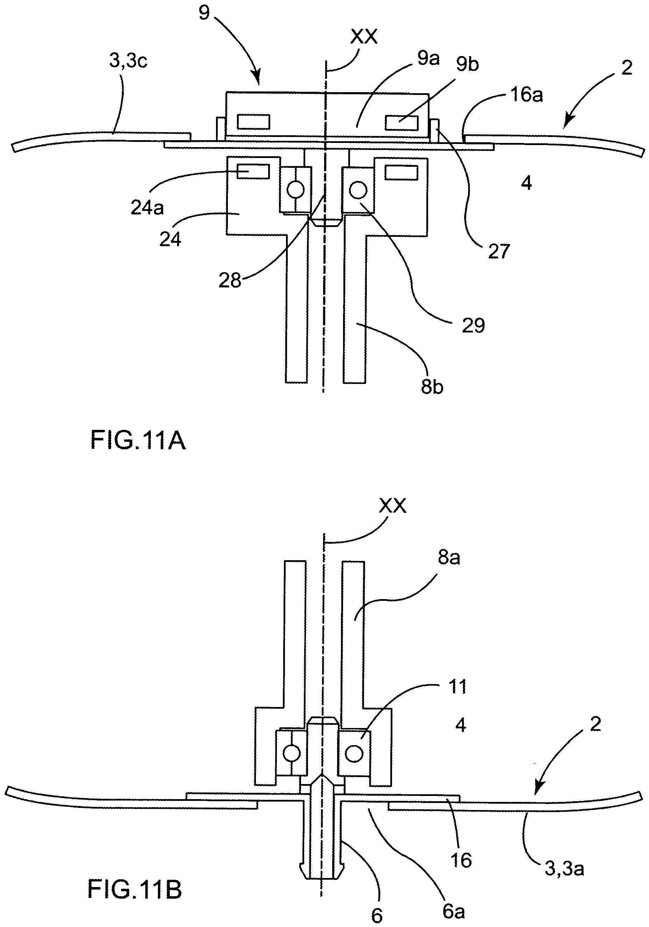

On se réfère maintenant plus spécialement aux figures 10 et 11A.

La flasque supérieure 16a est associée à un moyen moteur 9 magnétique, positionné à l'intérieur de l'accouplement d'entraînement 27, de manière à orienter les aimants d'entraînement 9b vers les aimants entraînés 24a. Il est prévu sur la face intérieure de la flasque supérieure 16a, un pivot 28 pour le maintien de l'arbre 8 à son extrémité supérieure 8b, autorisant son pivotement. Il est prévu également des paliers de butée 29 orientés verticalement entre le pivot 28 de la flasque supérieure 16a et l'extrémité supérieure 8b de l'arbre 8. De plus, le disque magnétique 24 de cette réalisation est formé sur l'extrémité supérieure terminale 8b de l'arbre 8, de manière à pouvoir constituer un arbre 8 en une seule pièce, si cela est souhaité.

Selon une autre seconde variante d'exécution possible (non représentée), le au moins un arbre 8 des moyens 7 de mélange est situé pour partie dans l'espace intérieur 4 et pour partie à l'extérieur du conteneur 2, une liaison tournante étanche étant prévue. Dans ce cas, le moyen moteur 9 d'entraînement à rotation de l'arbre 8 peut être à fonctionnement mécanique, un arbre rotatif menant, situé à l'extérieur du conteneur 2, coopérant fonctionnellement avec la partie extérieure du au moins un arbre 8.

Selon une autre première variante d'exécution (figures 1 et 2), les moyens 7 de mélange comportent un unique arbre 8 dressé. On entend par « dressé », le fait que l'arbre 8 s'étend généralement dans une direction haut-bas ou verticale.

Selon une autre seconde variante d'exécution (figure 3), les moyens 7 de mélange comportent plusieurs - ici trois - arbres 8_{α}, 8_{β} et 8_{γ}, dressés, d'axes substantiellement parallèles entre eux et tous attenants à la partie inférieure 3a de la paroi 3 du conteneur 2. Ces arbres 8_{α}, 8_{β} et 8_{γ} sont aptes à entraîner à rotation chacun au moins un organe de mélange 10. Le récipient-mélangeur 1 comporte dans cette variante d'exécution plusieurs organes de mélange 10_{α,} 10_{β} et 10_{γ}.

Selon une autre première variante (figures 1 et 2), un arbre 8 des moyens 7 de mélange supporte et entraîne un unique organe de mélange 10 situé en une unique localisation axiale sur l'arbre 8.

Selon une autre seconde variante (figure 3), un arbre 8 des moyens 7 de mélange - ici l'arbre 8_{α} - supporte et entraîne plusieurs organes de mélange 10_{δ} et 10_{ε} situés en une pluralité de localisations axiales sur l'arbre 8, 8_{α}.

Un organe de mélange 10 peut se présenter sous la forme d'une hélice ayant un moyeu supportant plusieurs pales.

Comme il a été dit précédemment, un organe de mélange 10 est substantiellement espacé de la partie inférieure 3**a** de la paroi 3 du conteneur 2, du palier inférieur 11 et du au moins un élément étendu 15a de distribution. Selon une réalisation, cet espacement ou distance est de l'ordre d'au moins le quart de l'écartement entre la partie inférieure 3a et la partie supérieure 3c de la paroi 3 du conteneur 2. En particulier, cet espacement ou distance est de l'ordre d'au moins le tiers de l'écartement entre la partie inférieure 3a et la partie supérieure 3c de la paroi 3 du conteneur 2.

On décrit maintenant plus spécialement différentes variantes d'exécution du port combiné vidange/palier 6+11.

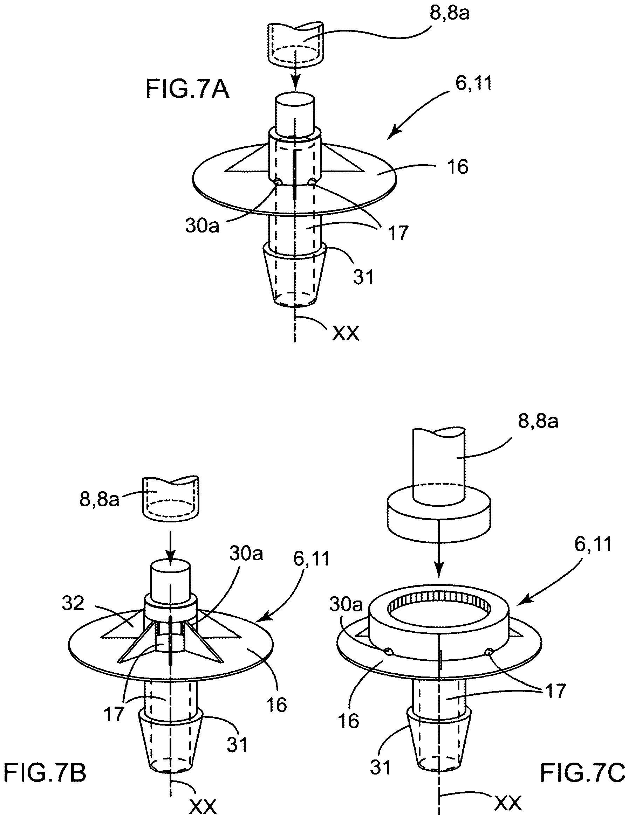

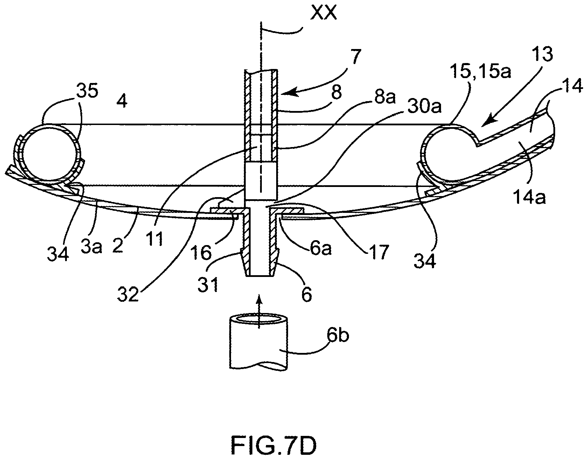

La flasque inférieure 16 associée à la partie inférieure 8a de l'arbre 8 et faisant partie du récipient-mélangeur 1, est plus particulièrement représenté sur les figures 7A, 7B et 7C selon plusieurs variantes d'exécution. Dans chacune de ces variantes, la flasque inférieure 16 est formé d'une matière sensiblement rigide, de préférence une matière plastique rigide, en forme de paroi ou de plaquette raccordée au conteneur flexible 2 dans son axe XX, au centre de la partie inférieure 3a, laquelle est la partie la plus basse du conteneur 2 dont la forme est incurvée, comme il a été dit. 4. Cette flasque 16 peut être raccordée au conteneur flexible 2 de toute manière appropriée de façon à former un joint rigide et hermétique entre les matières respectives, rigide et flexible.

La partie supérieure de la flasque inférieure 16, qui se trouve dans l'espace intérieur 4 du conteneur 2, inclut le palier inférieur 11 qui forme un moyen de raccordement qui coopère avec la partie inférieure 8a de l'arbre 8.

Le palier 11 peut être un palier mâle en forme de tourillon, tel que représenté sur les figures 7A et 7B, inséré dans une cavité ouverte ménagée dans la partie inférieure 8a de l'arbre 8. Le palier 11 peut être un palier femelle en forme de couronne, tel que représenté sur la figure 7C, dans la cavité de laquelle est insérée la partie inférieure 8a de l'arbre 8.

Il est préféré que l'arbre 8 s'adapte sur le palier 11 avec un frottement minimal, de sorte que l'arbre 8 peut tourner librement sur le palier 11. A cet effet, on prévoit d'inclure possiblement un palier de butée (non représenté) entre la partie inférieure 8a de l'arbre 8 et le palier 11, ou il peut être prévu des paliers lisses, à billes ou à rouleaux.

Si cela est souhaité, un cliquet (non représenté), qui ne compromet pas significativement la rotation de l'arbre 8 sur le palier 11, peut être prévu afin de maintenir l'arbre 8 sur le palier 11.

Il est entendu que le palier inférieur 11 et le palier supérieur 11a, lorsqu'il est prévu, peuvent avoir des structures analogues à celles plus spécialement décrites précédemment. Comme indiqué, selon les cas, le palier supérieur 11a n'assure que la fonction de palier ou bien il est combiné dans un port introduction/palier 5/11a.

Comme il a été indiqué, à la flasque inférieure 16 est associé un port de vidange 6 avec un orifice de vidange 6a ménagé dans le conteneur 2. La flasque 16 est donc pourvue d'un passage de vidange 17 en communication fluidique d'un côté avec l'espace intérieur 4 du conteneur 2 et de l'autre avec l'extérieur du conteneur 2 via le port de vidange 6 proprement dit, ici en forme de tronçon de tube comportant une partie extrême comportant une saillie périphérique extérieure en forme de dent de requin, permettant la fixation de la partie extrême d'un conduit de vidange 6b.

Le passage de vidange 17 comporte une ou plusieurs ouvertures 30a assurant la communication avec l'espace intérieur 4. Ces ouvertures 30a sont disposées en fonction de la structure du palier 11.

Dans la variante de la figure 7A, il est prévu une pluralité d'ouvertures 30a radiales ou sensiblement radiales, ménagées à la base du palier 11 en forme de tourillon, réparties autour, et ces ouvertures 30a débouchent dans le port de vidange 6.

Dans la variante de la figure 7B, il est prévu une ouverture 30a unique, axiale, ménagée axialement en dessous de la base du palier en forme de tourillon 11, laquelle base est surélevée par rapport à la flasque 16 au moyen d'entretoises 32 disposées radialement et axialement, un espace étant ainsi ménage entre cette base et la flasque 16.

Dans la variante de la figure 7C, il est prévu une pluralité d'ouvertures 30a radiales ou sensiblement radiales, ménagées à la base du palier 11 en forme de couronne, réparties autour, et ces ouvertures 30a débouchent dans le port de vidange 6.

On décrit maintenant plus spécialement différentes variantes d'exécution des moyens 13 d'aération.

Le au moins un élément tubulaire 14a d'amenée de gaz d'aération s'étend dans l'espace intérieur 4 du conteneur, en étant substantiellement maintenu attenant ou adjacent ou contre la face intérieure de la paroi 3 du conteneur 2, de manière à éviter que l'élément tubulaire 14a ne divague dans le conteneur, alors que le contenu de celui-ci est agité par les moyens 7 de mélange, au risque d'interférer avec ceux-ci.

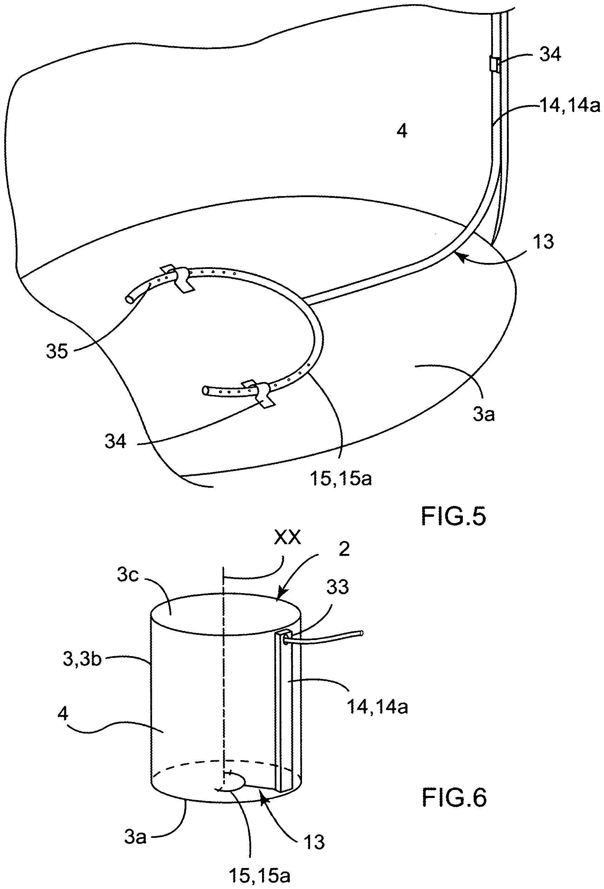

A cet effet, selon les différentes variantes d'exécution envisageables, le au moins un élément tubulaire 14a d'amenée de gaz d'aération est au moins pour partie structurellement distinct de la paroi 3 du conteneur 2 et maintenue à elle par collage, soudage ou au moyen de pièces de maintien 34, apportées comme il est représenté sur les figures 1 à 3, et/ou au moins pour partie structurellement partie intégrante de la paroi 3 du conteneur 2 comme il est représenté sur la figure 6.

En référence aux figures 1 à 3, des exemples typiques de pièces de maintien 34 sont des bandes d'adhésif, des brides, des cavaliers ou analogue disposées de place en place le long de l'élément tubulaire 14a d'amenée de gaz d'aération.

En référence à la figure 6, l'élément tubulaire 14a d'amenée est ici prévu un manchon. Ce manchon est construit en une longueur de matière, de préférence, identique à celle de la face intérieure de la paroi 3 du conteneur flexible 2, soudée ou fixée autrement sur ses côtés longitudinaux sur la face intérieure de la paroi 3 en étant placé à l'intérieur du conteneur flexible 2. La zone tubulaire comprise entre ce manchon et la face intérieure de la paroi 3 du conteneur flexible 2 achemine le gaz depuis l'extérieur du récipient-mélangeur jusqu'aux moyens 15 de distribution de gaz d'aération.

En amont (arrivée du gaz d'aération), le au moins un élément tubulaire 14a d'amenée de gaz d'aération traverse la paroi 3 du conteneur dans la partie supérieure 3c, par une liaison étanche 33. Cette disposition constructive permet, d'une part, de libérer la zone située au dessous de la partie inférieure 3a qui n'est pas encombrée par cet élément tubulaire 14a et, d'autre part, de ne pas être obligé de prévoir dans la partie inférieure 3a de la paroi 3 du conteneur 2, une ouverture de passage pour l'élément tubulaire 14a.

D'autre part, le au moins un élément étendu 15a de distribution de gaz d'aération est maintenu attenant ou adjacent à la face intérieure de la partie inférieure 3a de la paroi 3 du conteneur 2.

Selon les différentes variantes d'exécution envisageables, le au moins un élément étendu 15a de distribution de gaz d'aération est, au moins pour partie, structurellement distinct de la paroi 3 du conteneur 2 et maintenu à elle par collage, soudage ou au moyen de pièces de maintien rapportées et/ou au moins pour partie, structurellement partie intégrante de la paroi 3 du conteneur 2. En tout état de cause, le au moins un élément étendu 15a de distribution de gaz d'aération ne traverse pas la paroi 3 du conteneur 2. De telles pièces de maintien rapportées, lorsqu'elles sont prévues, peuvent être identiques ou analogues à celles utilisées pour l'élément tubulaire 14a d'amenée.

Bien entendu, la variante d'exécution du au moins un élément tubulaire 14a d'amenée de gaz d'aération est en adéquation avec celle du au moins un élément étendu 15a de distribution de gaz d'aération.

Le au moins un élément tubulaire 14a d'amenée de gaz d'aération s'étend, depuis l'amont vers l'aval, depuis l'extérieur vers l'intérieur du conteneur 2 et l'élément étendu 15a de distribution de gaz d'aération, par la liaison étanche 33 dans la partie supérieure 3c, puis axialement le long de la face intérieure de la partie latérale 3b jusqu'à la face intérieure de la partie inférieure 3a, puis radialement ou sensiblement radialement sur la face intérieure de la partie inférieure 3a, jusqu'à le au moins un élément étendu 15a de distribution de gaz d'aération attenant ou adjacent à la même face intérieure de la partie inférieure 3a de la paroi 3.

Le au moins un élément étendu 15a de distribution de gaz d'aération est du type comportant une paroi pourvue d'une pluralité de trous 35, répartis sur cette paroi. L'élément tubulaire 14a d'amenée de gaz d'aération débouche en communication fluidique d'un côté de cette paroi 15a, alors que l'autre côté de cette paroi 15a est située dans l'espace intérieur 4. Les trous 35 sont aptes au passage des bulles du gaz d'aération provenant des moyens 14 d'amenée vers l'espace intérieur 4.

Les trous 35 peuvent faire l'objet de différentes variantes d'exécution.

Selon une variante d'exécution possible, les trous 35 de la pluralité de trous 35 sont orientés avec différents axes d'inclinaison sur l'axe XX vertical.

Selon d'autres variantes d'exécution possible, les trous 35 de la pluralité de trous 35 sont soit de même taille soit de tailles différentes.

Ainsi, le débit du gaz d'aération sortant des trous 35 et l'orientation de sortie des bulles de gaz en aval de la paroi de l'élément étendu 15a de distribution de gaz d'aération peuvent être adaptés en fonction des besoins.

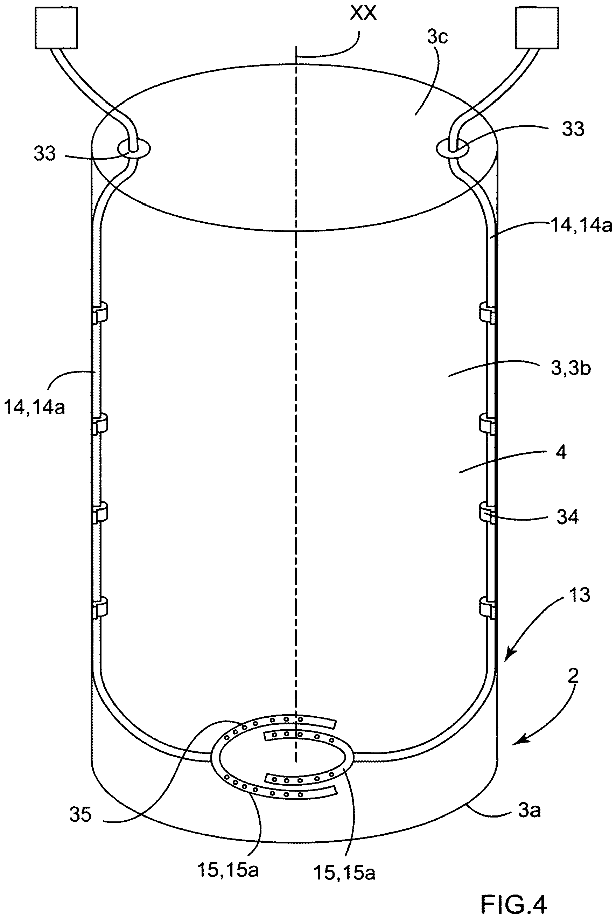

Ces variantes d'exécution s'appliquent aussi bien à une variante d'exécution selon laquelle les moyens 13 d'aération comportent un unique ensemble de moyens 14 d'amenée de gaz d'aération et de moyens 15 de distribution de gaz d'aération (non représenté), et à une variante selon laquelle les moyens 13 d'aération comportent plusieurs ensembles distincts de moyens 14 d'amenée d'un ou de plusieurs gaz d'aération et de moyens 15 de distribution du ou des gaz d'aération, comme il est représenté sur les figures 1, 2, 3, 4.

Une telle disposition constructive à plusieurs ensembles distincts de moyens 14 d'amenée et de moyens 15 de distribution d'un ou de plusieurs gaz d'aération est particulièrement bien adaptée au cas où le procédé exige l'aération avec plusieurs gaz, par exemple de l'aération avec de l'oxygène et de l'aération avec du gaz carbonique.

Dans le cas d'une disposition à plusieurs ensembles distincts de moyens 14 d'amenée et de moyens 15 de distribution d'un ou de plusieurs gaz d'aération, les différents moyens 15 de distribution de la pluralité de moyens 15 de distribution ont soit les mêmes caractéristiques soit ont des caractéristiques différentes associées au type ou au volume de gaz introduit dans le contenu C. Ces différentes caractéristiques peuvent inclure, mais de façon non limitative, la dimension et le nombre de trous 35. Selon une variante d'exécution, les moyens 13 d'aération sont tels qu'à un unique élément tubulaire 14a d'amenée est associé un unique élément étendu 15a de distribution. Selon d'autres variantes d'exécution, à un unique élément tubulaire 14a d'amenée sont associés plusieurs éléments étendus 15a de distribution ou, inversement qu'à plusieurs éléments tubulaires 14a d'amenée est associé un unique élément étendu 15a de distribution.

Le au moins un élément étendu 15a de distribution de gaz d'aération peut faire l'objet de différentes variantes d'exécution.

Dans une variante d'exécution, le au moins un élément étendu 15a de distribution de gaz d'aération a, en élévation, une forme générale annulaire ou pseudo annulaire, laquelle a, en section droite transversale, une forme générale qui peut-être circulaire, ou pseudo-circulaire, ou elliptique ou pseudo-elliptique. Un tel au moins un élément annulaire 15a est en communication fluidique avec l'élément tubulaire d'amenée 14a.

Selon une variante, à l'élément annulaire 15a est associé au moins un élément transversal, notamment radial, ce qui permet une distribution de gaz plus répartie.

Selon les cas, l'élément annulaire 15a est en forme d'anneau complet fermé sur lui-même, en communication fluidique circulaire continue ou non, ou est en forme anneau incomplet ouvert par rapport à lui-même. Dans une réalisation, l'ouverture d'angle d'un tel anneau ouvert est comprise entre environ 180° et 270°.

Dans une variante d'exécution, un tel élément annulaire 15a est sensiblement centré sur le port combiné vidange/palier.

Dans une variante d'exécution, les moyens 13 d'aération comportent un unique ensemble de moyens 14 d'amenée de gaz d'aération et de moyens 15 de distribution de gaz d'aération. Dans une autre variante d'exécution, les moyens 13 d'aération comportent plusieurs ensembles distincts de moyens 14 d'amenée d'un ou de plusieurs gaz d'aération et de moyens 15 de distribution du ou des gaz d'aération.

Un tel ensemble 14+15 de moyens 13 d'aération peut faire l'objet de différentes variantes d'exécution, en ce que ledit ensemble comporte soit un seul élément tubulaire 14a d'amenée de gaz d'aération communiquant avec un seul élément annulaire 15a de distribution de gaz d'aération, soit un seul élément tubulaire 14a communiquant avec plusieurs éléments annulaire 15a, soit plusieurs éléments tubulaires 14a communiquant avec un seul élément annulaire 15a, soit encore plusieurs éléments tubulaires 14a communiquant avec plusieurs éléments étendus annulaire 15a.

D'autre part, dans le cas où un tel ensemble 14+15 comporte plusieurs éléments annulaire 15a de distribution de gaz d'aération distincts, il est possible, dans une variante d'exécution, que ces - ou certains de ces - éléments annulaire 15*a* soient situés en plusieurs localisations radiales dans l'espace intérieur 4 et vers la partie inférieure 3a de la paroi 3 du conteneur 2. Dans ce cas, de telles localisations radiales peuvent, dans une variante d'exécution, être substantiellement espacées les unes des autres, radialement par rapport à l'axe XX entre le port vidange/palier 6+11 jusqu'au voisinage de la partie latérale 3b de la paroi 3 du conteneur 2.

Avec une telle disposition, l'aération est particulièrement bien répartie, au niveau de sa distribution.

Comme il a été dit précédemment, un élément annulaire 15a de distribution de gaz d'aération est substantiellement espacé radialement du port vidange/palier 6+11. Selon une réalisation, cet espacement ou distance est de l'ordre d'au moins le cinquième du diamètre de la partie inférieure de la paroi 3 du conteneur 2.

Aux moyens 13 d'aération qui viennent d'être décrits peuvent être associés fonctionnellement au moins un port d'évacuation de gaz d'aération 36 coopérant avec au moins un orifice d'évacuation ménagé dans la partie supérieure 3c de la paroi 3 du conteneur 2. Un tel port d'évacuation de gaz d'aération 36 peut être pourvu d'une valve anti-retour, empêchant l'introduction dans le conteneur 2 de fluides ou de contaminants non souhaités ou indésirables. Un tel port d'évacuation de gaz d'aération 36 permet d'évacuer du conteneur 2, vers l'extérieur, le gaz qui n'a pas été mélangé dans le contenu du conteneur 2. Un tel port d'évacuation de gaz d'aération 36 peut être en communication fluidique avec l'entrée de gaz d'aération, en vue du recyclage.

Le récipient-mélangeur 1 peut, dans certaines réalisations, comporter également un ou plusieurs ports 37 de montage par exemple d'un moyen fonctionnel, apte à assurer le maintien d'un organe fonctionnel 38 tel que typiquement la collecte ou la mesure de données, la prise d'échantillon aux fins d'analyse.

## Revendications

1. Récipient-mélangeur (1) destiné à recevoir un contenu biopharmaceutique (C) en vue de son mélange, comprenant:
■ un conteneur flexible (2), comportant :
○ une paroi (3) ayant une partie inférieure (3a), une partie latérale (3b) et une partie supérieure (3c), délimitant un espace intérieur (4) apte à recevoir une certaine quantité du contenu (C),
○ un ou plusieurs ports d'introduction (5) dans le conteneur (2) du contenu (C) ou de composants du contenu (C), coopérant avec un ou plusieurs orifices d'introduction (5a) ménagés dans le conteneur (2),
○ au moins un port de vidange (6) (6) du contenu (C) coopérant avec au moins un orifice de vidange (6) (6a),
■ des moyens de mélange (7) du contenu (C), comportant :
○ au moins un arbre (8) dressé, apte à être entraîné à rotation par des moyens moteurs (9) et à entraîner à rotation au moins un organe de mélange (10),
○ au moins un palier inférieur (11), adjacent à la partie inférieure (3a) de la paroi (3) du conteneur (2), avec lequel coopère la partie inférieure (8a) de l'arbre (8),
○ au moins un organe de mélange (10), apte à agiter le contenu (C), situé dans l'espace intérieur (4),
■ des moyens d'aération (13) aptes à délivrer au contenu (C) une certaine quantité de gaz d'aération, comportant :
○ des moyens (14) d'amenée de gaz d'aération ayant au moins un élément tubulaire (14a) s'étendant avec communication fluidique depuis l'extérieur du conteneur (2) jusqu'aux moyens de distribution (15),
○ des moyens de distribution (15) de gaz d'aération comprenant au moins un élément étendu de distribution (15a) dont la paroi laisse passer des bulles du gaz d'aération provenant des moyens d'amenée, situé dans l'espace intérieur (4) vers la partie inférieure (3a) de la paroi (3) du conteneur (2),
**caractérisé par le fait que** :
■ il comporte au moins un port combiné vidange (6)/palier inférieur (11) ayant une flasque (16a) rigide :
○ pourvue d'un passage de vidange (17) en communication fluidique d'un côté avec l'espace intérieur (4) et de l'autre avec l'extérieur du conteneur (2),
○ fixée de façon rigide et étanche à la partie inférieure (3a) de la paroi (3) du conteneur (2) autour de l'orifice de vidange (6), le passage de vidange (17) et l'ouverture de vidange (6) étant en communication fluidique,
○ supportant du côté intérieur un palier inférieur (11) situé dans l'espace intérieur (4), adjacent au passage de vidange (17) sans empêcher la communication fluidique entre le passage de vidange (17) et l'ouverture de vidange (6),
■ le au moins un élément étendu de distribution (15a) de gaz d'aération est espacé radialement du port vidange (6)/palier (11),
■ le au moins un élément tubulaire (14a) d'amenée de gaz d'aération s'étend à partir de l'élément étendu de distribution (15a), dans l'espace intérieur (4), le long de la face intérieure de la partie inférieure (3a) et de la partie latérale (3b) de la paroi (3) du conteneur (2) et s'étend à l'extérieur du conteneur (2) à partir de la - ou du voisinage de la - partie supérieure (3c) de la paroi (3) du conteneur (2),
■ au moins un organe de mélange (10) est espacé de la partie inférieure (3a) de la paroi (3) du conteneur (2), du palier inférieur (11) et du au moins un élément étendu de distribution (15a) de gaz d'aération,
de manière que les bulles du gaz d'aération distribuées depuis le au moins un élément étendu de distribution (15a) du gaz d'aération, soient réparties dans le contenu (C) par une première répartition dans la zone inférieure de l'espace intérieur (4) adjacente à la partie inférieure (3a) de la paroi (3) du conteneur (2), par le au moins un élément étendu de distribution (15a) du gaz de distribution et une seconde répartition par le au moins un organe de mélange (10) dans l'ensemble de l'espace intérieur (4) du conteneur (2).

2. Récipient-mélangeur (1) selon la revendication 1, **caractérisé par le fait que** le au moins un arbre (8) des moyens de mélange (7) coopère soit avec un unique palier, le palier inférieur (11), le moyen moteur (9) d'entraînement à rotation de l'arbre (8) étant situé vers la partie inférieure (3a) de la paroi (3) du conteneur (2) soit avec deux paliers, sa partie inférieure (8a) avec le palier inférieur (11) et sa partie supérieure (8b) avec un palier supérieur (11a) adjacent à la partie supérieure.

3. Récipient-mélangeur (1) selon la revendication 2, **caractérisé par le fait qu'**il comporte au moins un port combiné introduction (5)/palier supérieur (11a) ayant une flasque (16a) rigide :
o pourvue d'un passage d'introduction du contenu (C) ou de composants du contenu (C) en communication fluidique d'un côté avec l'espace intérieur (4) et de l'autre avec l'extérieur du conteneur (2),
o fixée de façon rigide et étanche à la partie supérieure de la paroi (3) du conteneur (2) autour de l'orifice d'introduction, le passage d'introduction et l'ouverture d'introduction étant en communication fluidique,
o supportant du côté intérieur le palier supérieur (11a) situé dans l'espace intérieur (4), adjacent au passage d'introduction sans empêcher la communication fluidique entre le passage d'introduction et l'ouverture d'introduction.

4. Récipient-mélangeur (1) selon l'une quelconque des revendications 1 à 3, **caractérisé par le fait que** le au moins un arbre (8) des moyens de mélange (7) est situé tout entier dans l'espace intérieur (4), le moyen moteur (9) d'entraînement à rotation de l'arbre (8) étant à fonctionnement magnétique, un disque rotatif menant à pôles magnétiques (9a), situé à l'extérieur du conteneur (2), coopérant fonctionnellement avec un disque rotatif mené à pôles magnétiques, fixé sur le au moins un arbre (8) à proximité magnétique du disque rotatif menant (9a).

5. Récipient-mélangeur (1) selon l'une quelconque des revendications 1 à 3, **caractérisé par le fait que** le au moins un arbre (8) des moyens de mélange (7) est situé pour partie dans l'espace intérieur (4) et pour partie à l'extérieur du conteneur (2), le moyen moteur (9) d'entraînement à rotation de l'arbre (8) étant à fonctionnement mécanique, un arbre rotatif menant, situé à l'extérieur du conteneur (2), coopérant fonctionnellement avec la partie extérieure du au moins un arbre (8).

6. Récipient-mélangeur (1) selon l'une quelconque des revendications 1 à 5, **caractérisé par le fait que** les moyens de mélange (7) comportent soit un unique arbre (8) dressé soit plusieurs arbres (8) dressés d'axes parallèles, aptes à entraîner à rotation chacun au moins un organe de mélange (10).

7. Récipient-mélangeur (1) selon l'une quelconque des revendications 1 à 6, **caractérisé par le fait qu'**un arbre (8) des moyens de mélange (7) supporte et entraîne soit un unique organe de mélange (10) situé en une unique localisation axiale sur l'arbre (8) soit plusieurs organes de mélange situés en une pluralité de localisations axiales sur l'arbre (8).

8. Récipient-mélangeur (1) selon l'une quelconque des revendications 1 à 7, **caractérisé par le fait qu'**un organe de mélange (10) est espacé de la partie inférieure (3a) de la paroi (3) du conteneur (2), du palier inférieur (11) et du au moins un élément étendu de distribution (15a), d'une distance de l'ordre d'au moins le quart de l'écartement entre la partie inférieure (3a) et la partie supérieure (3c) de la paroi (3) du conteneur (2).

9. Récipient-mélangeur (1) selon l'une quelconque des revendications 1 à 8, **caractérisé par le fait qu'**un organe de mélange (10) est espacé de la partie inférieure (3a) de la paroi (3) du conteneur (2), du palier inférieur (11) et du au moins un élément étendu de distribution (15a), d'une distance de l'ordre d'au moins le tiers de l'écartement entre la partie inférieure (3a) et la partie supérieure (3c) de la paroi (3) du conteneur (2).

10. Récipient-mélangeur (1) selon l'une quelconque des revendications 1 à 9, **caractérisé par le fait que** le au moins un élément tubulaire (14a) d'amenée de gaz d'aération s'étend dans l'espace intérieur (4), en étant maintenu attenant ou adjacent à la face intérieure de la paroi (3) du conteneur (2).

11. Récipient-mélangeur (1) selon l'une quelconque des revendications 1 à 10, **caractérisé par le fait que** le au moins un élément tubulaire (14a) d'amenée de gaz d'aération est au moins pour partie soit structurellement distinct de la paroi (3) du conteneur (2) et maintenue à elle par collage, soudage ou au moyen de pièces de maintien rapportées soit partie intégrante de la paroi (3) du conteneur (2).

12. Récipient-mélangeur (1) selon l'une quelconque des revendications 1 à 11, **caractérisé par le fait que** le au moins un élément tubulaire (14a) d'amenée de gaz d'aération traverse la paroi (3) du conteneur (2) par une liaison étanche.

13. Récipient-mélangeur (1) selon l'une quelconque des revendications 1 à 12, **caractérisé par le fait que** le au moins un élément tubulaire (14a) d'amenée de gaz d'aération traverse la paroi (3) du conteneur (2) dans la partie supérieure (3c).

14. Récipient-mélangeur (1) selon l'une quelconque des revendications 1 à 13, **caractérisé par le fait que** le au moins un élément étendu de distribution (15a) de gaz d'aération est maintenu attenant ou adjacent à la face intérieure de la partie inférieure (3a) de la paroi (3) du conteneur (2).

15. Récipient-mélangeur (1) selon l'une quelconque des revendications 1 à 14, **caractérisé par le fait que** le au moins un élément étendu de distribution (15a) de gaz d'aération est, au moins pour partie, structurellement distinct de la paroi (3) du conteneur (2) et maintenu à elle par collage, soudage ou au moyen de pièces de maintien rapportées.

16. Récipient-mélangeur (1) selon l'une quelconque des revendications 1 à 15, **caractérisé par le fait que** le au moins un élément étendu de distribution (15a) de gaz d'aération est, au moins pour partie, structurellement partie intégrante de la paroi (3) du conteneur (2).

17. Récipient-mélangeur (1) selon l'une quelconque des revendications 1 à 16, **caractérisé par le fait que** le au moins un élément tubulaire d'amenée (14a) s'étend, depuis l'amont vers l'aval, depuis l'extérieur vers l'intérieur du conteneur (2) et l'élément étendu (15a) de distribution de gaz d'aération est en adéquation avec le au moins un élément tubulaire (14a) d'amenée.

18. Récipient-mélangeur (1) selon l'une quelconque des revendications 1 à 17, **caractérisé par le fait que** le au moins un élément étendu de distribution (15a) de gaz d'aération comporte une paroi (3) pourvue d'une pluralité de trous répartis aptes au passage des bulles du gaz d'aération provenant des moyens d'amenée (14).

19. Récipient-mélangeur (1) selon la revendication 18, **caractérisé par le fait que** la pluralité de trous aptes au passage des bulles du gaz d'aération provenant des moyens d'amenée (14) est orientée avec différents axes d'inclinaison sur la verticale.

20. Récipient-mélangeur (1) selon l'une quelconque des revendications 18 et 19, **caractérisé par le fait que** les trous de la pluralité de trous sont soit de même taille soit de tailles différentes.

21. Récipient-mélangeur (1) selon l'une quelconque des revendications 1 à 20, **caractérisé par le fait que** le au moins un élément étendu de distribution (15a) de gaz d'aération a, en section droite transversale, une forme circulaire, ou pseudo-circulaire, ou elliptique ou pseudo-elliptique.

22. Récipient-mélangeur (1) selon l'une quelconque des revendications 1 à 21, **caractérisé par le fait que** le au moins un élément étendu de distribution (15a) de gaz d'aération comprend au moins un anneau complet fermé sur lui-même, en communication circulaire continue ou non et/ou au moins un anneau incomplet ouvert par rapport à lui-même, notamment ayant une ouverture d'angle comprise entre environ 180° et 270°.

23. Récipient-mélangeur (1) selon l'une quelconque des revendications 1 à 22, **caractérisé par le fait que** les moyens d'aération (13) comportent un unique ensemble de moyens (14) d'amenée de gaz d'aération et de moyens de distribution (15) de gaz d'aération ou plusieurs ensembles distincts de moyens d'amenée d'un ou de plusieurs gaz d'aération et de moyens de distribution (15) du ou des gaz d'aération.

24. Récipient-mélangeur (1) selon la revendication 23, **caractérisé par le fait qu'**un ensemble de moyens d'aération (13) comporte un seul élément tubulaire (14a) d'amenée de gaz d'aération communiquant avec un seul élément étendu de distribution (15a) de gaz d'aération, ou un seul élément tubulaire (14a) d'amenée de gaz d'aération communiquant avec plusieurs éléments étendus de distribution (15a) de gaz d'aération, ou plusieurs éléments tubulaires (14a) d'amenée de gaz d'aération communiquant avec un seul élément étendu de distribution (15a) de gaz d'aération, ou plusieurs éléments tubulaires (14a) d'amenée de gaz d'aération communiquant avec plusieurs éléments étendus de distribution (15a) de gaz d'aération.

25. Récipient-mélangeur (1) selon l'une quelconque des revendications 1 à 24, dans le cas où il comporte plusieurs éléments étendus de distribution (15a) de gaz d'aération distincts, **caractérisé par le fait que** au moins certains des plusieurs éléments étendus de distribution (15a) de gaz d'aération sont situés en une pluralité de localisations radiales dans l'espace intérieur (4) vers la partie inférieure (3a) du conteneur (2), notamment sont espacés radialement du port vidange (6)/palier (11) jusqu'au voisinage de la partie latérale (3b) de la paroi (3) du conteneur (2).

26. Récipient-mélangeur (1) selon l'une quelconque des revendications 1 à 25, **caractérisé par le fait qu'**un élément étendu de distribution (15a) de gaz d'aération est espacé radialement du port vidange (6)/palier (11), d'une distance de l'ordre d'au moins le cinquième du diamètre de la partie inférieure (3a) de la paroi (3) du conteneur (2).

27. Récipient-mélangeur (1) selon l'une quelconque des revendications 1 à 26, **caractérisé par le fait que** ne saille sous la partie inférieure (3a) de la paroi (3) du conteneur (2) que la vidange (6) et, le cas échéant, le moyen moteur (9) d'entraînement des moyens de mélange (7) lorsqu'il est prévu en partie inférieure.

28. Récipient-mélangeur (1) selon l'une quelconque des revendications 1 à 27, **caractérisé par le fait qu'**il comporte également un ou plusieurs ports d'évacuation de gaz coopérant avec au moins un orifice d'évacuation ménagé dans la partie supérieure de la paroi (3) du conteneur (2), pourvu d'une valve anti-retour, empêchant l'introduction dans le conteneur (2) de fluides ou de contaminants non souhaités ou indésirables et/ou un ou plusieurs ports d'introduction (5), de vidange (6), de montage.

29. Récipient-mélangeur (1) selon l'une quelconque des revendications 1 à 28, **caractérisé par le fait que** le conteneur (2) est de grande capacité, pouvant aller jusqu'à 5.000 litres.

30. Récipient-mélangeur (1) selon l'une quelconque des revendications 1 à 29, **caractérisé par le fait qu'**il comporte également un dispositif rigide (18) extérieur de contention du conteneur (2) empli de son contenu (C), comprenant une paroi (19) de fond, une paroi (20) périphérique et une ouverture supérieure (21), délimitant un logement principal dans lequel est placé de façon amovible le conteneur flexible (2) dont la partie inférieure repose sur la paroi (19) de fond et dont la partie latérale vient s'appliquer, lorsque le conteneur (2) est empli de son contenu (C), contre la paroi (20) périphérique.

31. Récipient-mélangeur (1) selon la revendication 30, **caractérisé par le fait que** le dispositif rigide (18) extérieur de contention comporte également un logement secondaire (22) en dessous de la paroi (19) de fond de logement et de protection de la vidange (6) et, le cas échéant, du moyen moteur (9) d'entraînement des moyens de mélange (7) lorsqu'il est prévu en partie inférieure.

32. Récipient-mélangeur (1) selon l'une quelconque des revendications 30 et 31, **caractérisé par le fait que** le dispositif rigide (18) extérieur de contention comporte également des moyens de chauffage et le conteneur flexible (2) est en un matériau présentant une certaine conductivité thermique, de manière que la mise en oeuvre des moyens de chauffage permette le chauffage du contenu (C) ; et, le cas échéant, des moyens de contrôle de la température du conteneur (2) et des moyens de commande des moyens de chauffage.

33. Récipient-mélangeur (1) selon l'une quelconque des revendications 30 à 32, **caractérisé par le fait que** le conteneur (2) peut se trouver dans trois états extrêmes: un état désassemblé du dispositif rigide (18) extérieur de contention dans lequel le conteneur (2) peut être disposé de façon aplatie sur lui-même, un état assemblé au dispositif rigide (18) extérieur de contention dans lequel le conteneur (2), vide de contenu (C), est disposé dans le logement principal du dispositif de contention (18) en reposant sur la paroi (19) de fond, et un état assemblé au dispositif rigide (18) extérieur de contention dans lequel le conteneur (2), empli de son contenu (C), est disposé dans le logement principal du dispositif de contention (18) en reposant sur la paroi (19) de fond et en étant appliqué contre la paroi (20) périphérique.

34. Récipient-mélangeur (1) selon l'une quelconque des revendications 1 à 33, **caractérisé par le fait que** l'on y réalise une bioréaction, le récipient-mélangeur (1) étant un bioréacteur.

35. Procédé de mise en oeuvre d'un Récipient-mélangeur (1) selon l'une quelconque des revendications 1 à 34, dans lequel :
■ on dispose d'un tel récipient-mélangeur (1) dont le port de vidange (6) est obturé,
■ on dispose du contenu (C) ou des composants du contenu (C) destinés à être reçus dans le conteneur (2) du récipient-mélangeur (1) puis mélangé,
■ on introduit dans le conteneur (2) le contenu (C) ou des composants du contenu (C),
■ on met en oeuvre les moyens de mélange (7) pour agiter le contenu (C) du conteneur (2),
■ on met en oeuvre les moyens d'aération (13) pour délivrer au contenu (C) une certaine quantité de gaz d'aération, l'aération et le mélange étant réalisés au moins pour partie simultanément, et
■ on distribue les bulles du gaz d'aération depuis le au moins un élément étendu de distribution (15a) du gaz d'aération et on les répartit dans le contenu (C) par une première répartition dans la zone inférieure de l'espace intérieur (4) adjacente à la partie inférieure (3a) de la paroi (3) du conteneur (2), par le au moins un élément étendu de distribution (15a) du gaz d'aération et une seconde répartition par le au moins un organe de mélange (10) dans l'ensemble de l'espace intérieur (4) du conteneur (2).

36. Procédé selon la revendication 35, **caractérisé par le fait que** l'on introduit d'abord dans le conteneur (2) un composant ou une partie des composants du contenu (C), on met en oeuvre les moyens de mélange (7) et les moyens d'aération (13) et on introduit pour délivrer au contenu (C) une certaine quantité de gaz d'aération, et on introduit dans le conteneur (2) le ou les composants restant du contenu (C).

37. Procédé selon l'une quelconque des revendications 35 et 36, **caractérisé par le fait que** :
■ l'on part d'un récipient-mélangeur (1) dont le conteneur (2) est désassemblé d'un dispositif rigide (18) extérieur de contention, vide de contenu (C), et disposé de façon aplatie sur lui-même,
■ on assemble le conteneur (2) au dispositif rigide (18) extérieur de contention, en le disposant dans le logement principal de celui-ci, en reposant sur sa paroi (19) de fond,
■ on introduit alors dans le conteneur (2) le contenu (C) ou des composants du contenu (C).

38. Procédé selon l'une quelconque des revendications 35 à 37, **caractérisé par le fait que** l'on dispose sous la partie inférieure (3a) de la paroi (3) du conteneur (2) la vidange (6) le moyen moteur (9) d'entraînement des moyens de mélange (7) lorsqu'il est prévu en partie inférieure.

## Claims

1. A mixing vessel (1) intended for receiving biopharmaceutical content (C) to be mixed, including:
- a flexible container (2), including:
* a wall (3) having a lower part (3a), a side part (3b) and an upper part (3c) defining an inner space (4) adapted for receiving a certain quantity of the content (C),
* one or several ports for the introduction (5) into the container (2) of the content (C) or content (C) components, cooperating with one or several introduction holes (5a) arranged in the container (2),
* at least one content (C) drain port (6) (6) cooperating with at least one drain hole (6)(6a),
- content (C) mixing means (7), including:
* at least one set-up shaft (8) adapted for being rotated by engine means (9) and for rotating at least one mixing member (10),
* at least one lower bearing (11) adjoining the lower part (3a) of the wall (3) of the container (2), with which the lower part (8a) of the shaft (8) cooperates,
* at least one mixing member (10), adapted for stirring the content (C), located in the inner space (4),
- aeration means (13) adapted for delivering into the content (C) a certain quantity of aeration gas, including:
* means (14) for supplying aeration gas having at least one tubular element (14a) extending, with a fluid communication, from the outside of the container (2) up to the distribution means (15),
* aeration gas distribution means (15) including at least one extended distribution element (15a) the wall of which lets aeration gas bubbles coming from the supplying means, located in the inner space (4), toward the lower part (3a) of the wall (3) of the container (2),
**characterized in that**:
- it includes at least one drain port (6)/upper bearing (11) combined port provided with a rigid flange (16a):
* provided with an evacuation passage (17) in fluid communication on one side with the inner space (4) and on the other side with the outside of the container (2),
* rigidly and sealingly fixed to the lower part (3a) of the wall (3) of the container (2) about the drain port (6), with the evacuation passage (17) and the drain port (6) being in fluid communication,
* supporting, on the inner side a lower bearing (11) located in the inner space (4), adjoining the evacuation passage (17) without preventing the fluid communication between the evacuation passage (17) and the drain port (6),
- the at least one extended aeration gas distribution element (15a) is radially spaced from the drain port (6)/bearing (11),
- the at least one tubular aeration gas supplying element (14a) extends from the extended aeration gas distribution element (15a), in the inner space (4), along the inner face of the lower part (3a) and the side part (3b) of the wall (3) of the container (2) and extends outside the container (2) from the - or the vicinity of the - upper part (3c) of the wall (3) of the container (2),
- at least one mixing member (10) is spaced from the lower part (3a) of the wall (3) of the container (2), the lower bearing (11) and the at least one extended aeration gas distribution element (15a),
so that the aeration gas bubbles distributed from the at least one extended aeration gas distribution element (15a) are dispatched in the content (C) by a first dispatch in the lower area of the inner space (4) adjoining the lower part (3a) of the wall (3) of the container (2), through the at least one extended aeration gas distribution element (15a) and a second dispatch through the at least one mixing member (10) into the whole inner space (4) of the container (2).

2. A mixing vessel (1) according to claim 1, **characterized in that** the at least one shaft (8) of the mixing means (7) cooperates either with a single bearing, the lower bearing (11), with the engine means (9) rotating the shaft (8) being located toward the lower part (3a) of the wall (3) of the container (2), or with two bearings, the lower part (8a) thereof with the lower bearing (11) and the upper part (8b) thereof with an upper bearing (11a) adjoining the upper part.

3. A mixing vessel (1) according to claim 2, **characterized in that** it includes at least one introduction (5)/upper bearing (11a) combined port provided with a rigid flange (16a):
* provided with a passage for introducing the content (C) or the content (C) components in fluid communication on one side with the inner space (4) and on the other side with the outside of the container (2),
* rigidly and sealingly fixed to the upper part of the wall (3) of the container (2) about the introduction hole, with the introduction passage and the introduction opening being in fluid communication,
* supporting on the inner side the upper bearing (11a) located in the inner space (4), adjoining the introduction passage without preventing the fluid communication between the introduction passage and the introduction opening.

4. A mixing vessel (1) according to any one of claims 1 to 3, **characterized in that** the at least one shaft (8) of the mixing means (7) is totally located in the inner space (4), with the engine means (9) rotating the shaft (8) being magnetically operated, with a driving rotating disk provided with magnetic poles (9a) located outside the container (2), functionally cooperating with a driven rotating disk with magnetic poles, fixed on the at least one shaft (8) in magnetic vicinity with the driving rotating disk (9a).

5. A mixing vessel (1) according to any one of claims 1 to 3, **characterized in that** the at least one shaft (8) of the mixing means (7) is partly located in the inner space (4) and partly outside the container (2), with the engine means (9) for rotating the shaft (8) being mechanically operated, with a driving rotating shaft, located outside the container (2), functionally cooperating with the outside part of the at least one shaft (8) .

6. A mixing vessel (1) according to any one of claims 1 to 5, **characterized in that** the mixing means (7) include either a single set-up shaft (8) or several set-up shafts (8) with parallel axes, adapted for rotating, each, at least one mixing member (10).

7. A mixing vessel (1) according to any one of claims 1 to 6, **characterized in that** one shaft (8) of the mixing means (7) supports and drives either a single mixing member (10) located in a single axial location on the shaft (8) or several mixing members located in a plurality of axial locations on the shaft (8).

8. A mixing vessel (1) according to any one of claims 1 to 7, **characterized in that** one mixing member (10) is spaced from the lower part (3a) of the wall (3) of the container (2), from the lower bearing (11) and from the at least one extended aeration gas distribution element (15a), by a distance of approximately at least a quarter of the distance between the lower part (3a) and the upper part (3c) of the wall (3) of the container (2).

9. A mixing vessel (1) according to any one of claims 1 to 8, **characterized in that** one mixing member (10) is spaced from the lower part (3a) of the wall (3) of the container (2), from the lower bearing (11) and from the at least one extended aeration gas distribution element (15a), by a distance of approximately at least one third of the distance between the lower part (3a) and the upper part (3c) of the wall (3) of the container (2).

10. A mixing vessel (1) according to any one of claims 1 to 9, **characterized in that** the at least one tubular aeration gas supplying element (14a) extends in the inner space (4) while being held close to or adjoining the inner face of the wall (3) of the container (2).

11. A mixing vessel (1) according to any one of claims 1 to 10, **characterized in that** the at least one tubular aeration gas supplying element (14a) is at least partially either structurally distinct from the wall (3) of the container (2) and applied thereto by gluing, welding or using added holding parts, or an integral part of the wall (3) of the container (2).

12. A mixing vessel (1) according to any one of claims 1 to 11, **characterized in that** the at least one tubular aeration gas supplying element (14a) goes through the wall (3) of the container (2) by means of a sealed bushing.

13. A mixing vessel (1) according to any one of claims 1 to 12, **characterized in that** the at least one tubular aeration gas supplying element (14a) goes through the wall (3) of the container (2) in the upper part (3c).

14. A mixing vessel (1) according to any one of claims 1 to 13, **characterized in that** the at least one extended aeration gas distribution element (15a) is held close to or adjoining the inner face of the lower part (3a) of the wall (3) of the container (2).

15. A mixing vessel (1) according to any one of claims 1 to 14, **characterized in that** the at least one extended aeration gas distribution element (15a) is at least partially structurally distinct from the wall (3) of the container (2) and applied thereto by gluing, welding or using added holding parts.

16. A mixing vessel (1) according to any one of claims 1 to 15, **characterized in that** the at least one extended aeration gas distribution element (15a) is at least partially structurally integral with the wall (3) of the container (2).

17. A mixing vessel (1) according to any one of claims 1 to 16, **characterized in that** the at least one tubular aeration gas supplying element (14a) extends, upstream to downstream, from the outside to the inside of the container (2) and the extended aeration gas distribution element (15a) matches the at least one tubular aeration gas supplying element (14a).

18. A mixing vessel (1) according to any one of claims 1 to 17, **characterized in that** the at least one extended aeration gas distribution element (15a) includes a wall (3) provided with a plurality of dispatched holes adapted for the passage of the aeration gas bubbles coming from the supplying means (14).

19. A mixing vessel (1) according to claim 18, **characterized in that** the plurality of holes adapted for the passage of the aeration gas bubbles coming from the supplying means (14) is oriented with various inclination axes along the vertical.

20. A mixing vessel (1) according to any one of claims 18 and 19, **characterized in that** the holes among the plurality of holes have the same size or different sizes.

21. A mixing vessel (1) according to any one of claims 1 to 20, **characterized in that** the at least one extended aeration gas distribution element (15a) has, in cross-section, a circular, or pseudo-circular, or elliptic, or pseudo-elliptic shape.

22. A mixing vessel (1) according to any one of claims 1 to 21, **characterized in that** the at least one extended aeration gas distribution element (15a) includes at least one complete closed loop, in continuous or not circular communication and/or at least one incomplete open loop, more particularly having an angle opening between approximately 180° and 270°.

23. A mixing vessel (1) according to any one of claims 1 to 22, **characterized in that** the aeration means (13) include only one set of aeration gas supplying means (14) and aeration gas distribution means (15) or several distinct sets of means for supplying one or several aeration gas and means for distributing (15) the aeration gas.

24. A mixing vessel (1) according to claim 23, **characterized in that** a set of aeration means (13) includes only one tubular aeration gas supplying element (14a) communicating with only one extended aeration gas distribution element (15a), or only one tubular aeration gas supplying element (14a) communicating with several extended aeration gas distribution elements (15a), or several tubular aeration gas supplying elements (14a) communicating with only one extended aeration gas distribution element (15a), or several tubular aeration gas supplying elements (14a) communication with several extended aeration gas distribution elements (15a).

25. A mixing vessel (1) according to any one of claims 1 to 24, when it includes several distinct extended aeration gas distribution elements (15a) **characterized in that** at least some of the extended aeration gas distribution elements (15a) are located in a plurality of radial locations in the inner space (4) toward the lower part (3a) of the container (2), more particularly are radially spaced from the drain port (6)/bearing (11) up to the vicinity of the side part (3b) of the wall (3) of the container (2).

26. A mixing vessel (1) according to any one of claims 1 to 25, **characterized in that** an extended aeration gas distribution element (15a) is radially spaced from the drain port (6)/bearing (11) by a distance of approximately one fifth of the diameter of the lower part (3a) of the wall (3) of the container (2).

27. A mixing vessel (1) according to any one of claims 1 to 26, **characterized in that** only the drain port (6) protrudes under the lower part (3a) of the wall (3) of the container (2), and if need be, the engine means (9) for driving the mixing means (7) when it is provided in the lower part.

28. A mixing vessel (1) according to any one of claims 1 to 27, **characterized in that** it also includes one or several gas discharge port(s) cooperating with at least one discharge hole arranged in the upper part of the wall (3) of the container (2), provided with a check valve, preventing the introduction into the container (2) of non desired or unwanted fluids or contaminants and/or one or several ports for the introduction (5), draining (6) or mounting.

29. A mixing vessel (1) according to any one of claims 1 to 28, **characterized in that** the content (2) has a large capacity, up to 5,000 liters.

30. A mixing vessel (1) according to any one of claims 1 to 29, **characterized in that** it also includes a rigid external retaining device (18) filled with its content (C), including a bottom wall (19), a peripheral wall (20) and an upper opening (21), defining a main housing wherein is removeably placed the flexible container (2) the lower part of which rests on the bottom wall (19) and the side part of which applies, when the container (2) is filled with its content (C), against the peripheral wall (20).

31. A mixing vessel (1) according to claim 30, **characterized in that** the rigid external retaining device (18) also includes a secondary housing (22) under the bottom wall (19) accommodating and protecting the drain port (6), and if need be, the engine means (9) for driving the mixing means (7) when it is provided in the lower part.

32. A mixing vessel (1) according to any one of claims 30 and 31, **characterized in that** the rigid external retaining device (18) also includes heating means and the flexible container (2) is made of a material having a certain thermal conductivity, so that implementing the heating means makes it possible to heat the content (C); and, if need be container (2) temperature control means and heating means control means.

33. A mixing vessel (1) according to any one of claims 30 to 32, **characterized in that** the container (2) can be in three extreme conditions: a disassembled condition of the rigid external retaining device (18) wherein the container (2) can be positioned flat, an assembled condition with the rigid external retaining device (18) wherein the container (2) containing no content (C) is placed in the main housing of the rigid external retaining device (18) by resting on the bottom wall (19), and an assembled condition with the rigid external retaining device (18) wherein the container (2) filled with its content (C) is positioned in the main housing of the rigid external retaining device (18) by resting on the bottom wall (19) and being applied against the peripheral wall (20).

34. A mixing vessel (1) according to any one of claims 1 to 33, **characterized in that** a bio-reaction is performed, with the mixing vessel (1) being a bioreactor.

35. A method for implementing a mixing vessel (1) according to any one of claims 1 to 34, wherein:
- such a mixing vessel (1) is available, the drain port (6) of which is plugged,
- the content (C) or the content (C) components intended to be received in the container (2) of the mixing vessel (1) is/are available and then mixed,
- the content (C) or content (C) components is/are introduced into the container (2),
- the mixing means (7) for stirring the content (C) of the container (2) are implemented,
- the aeration means (13) for delivering into the content (C) a certain quantity of aeration gas, with the aeration and the mixing being, at least partially carried out simultaneously, and
- the aeration gas bubbles are distributed from at least one extended aeration gas distribution element (15a) and are dispatched in the content (C) by a first dispatch into the lower area of the inner space (4) adjoining the lower part (3a) of the container (2) wall (3), by the at least one extended aeration gas distribution element (15a) and a second dispatch by the at least one mixing member (10) into the whole inner space (4) of the container (2).

36. A method according to claim 35, **characterized in that** a component or a part of the content (C) components are first introduced into the container (2), mixing means (7) and aeration means (13) are implemented and a certain quantity of aeration gas is introduced into the content (C), and the remaining component(s) of the content (C) is/are introduced into the container (2).

37. A method according to any one of claims 35 and 36, **characterized in that**:
- the starting element is a mixing vessel (1), the container (2) of which is disassembled from a rigid external retaining device (18) containing no content (C), and positioned flat,
- the container (2) is assembled with the rigid external retaining device (18) by positioning it in the main housing thereof, resting on the bottom wall (19) thereof,
- then the content (C) or content (C) components are introduced into the container (2).

38. A method according to any one of claims 35 to 37, **characterized in that** the drain port (6) of the engine means (9) for driving the mixing means (7) when it is provided in the lower part.

## Patentansprüche

1. Mischer-Behälter (1), der zur Aufnahme eines biopharmazeutischen Inhalts (C) zum Zweck seiner Vermischung bestimmt ist, umfassend:
* einen flexiblen Behälter (2), umfassend:
- eine Wand (3), die einen unteren Teil (3a), einen lateralen Teil (3b) und einen oberen Teil (3c) hat und die einen inneren Bereich (4) begrenzt, der geeignet ist, eine bestimmte Menge des Inhaltes (C) aufzunehmen
- einen oder mehrere Einführports (5) des Inhaltes (C) oder Komponenten des Inhaltes (C) in den Behälter (2), der / die mit einer oder mehreren Einführöffnungen (5a) zusammenwirkt / zusammenwirken, die im Behälter (2) ausgespart sind,
- wenigstens einen Entleerungsport (6) (6) des Behälters (2), der mit wenigstens einer Entleerungsöffnung (6) (6a) zusammenwirkt,
* Mischmittel (7) des Inhaltes (C), umfassend:
- wenigstens eine dressierte Welle (8), die geeignet ist, in Rotation von Motormitteln (9) angetrieben zu werden und wenigstens ein Mischorgan (10) in Rotation anzutreiben,
- wenigstens ein unteres Lager (11), das am unteren Teil (3a) der Wand (3) des Behälters (2) anliegt, mit dem der untere Teil (8a) der Welle (8) zusammenwirkt,
- wenigstens ein Mischorgan (10), das geeignet ist, den Inhalt (C) zu schütteln, der sich in dem inneren Raum (4) befindet,
* Belüftungsmittel (13), die geeignet sind, dem Behälter (C) eine bestimmte Menge Belüftungsgas zu liefern, umfassend:
- Zuführmittel (14) des Belüftungsgases mit wenigstens einem röhrenförmigen Element (14a), das sich mit fluidischer Kommunikation von der Außenseite des Behälters (2) bis zu den Verteilermitteln (15) erstreckt,
- Verteilermittel (15) des Belüftungsgases mit wenigstens einem erweiterten Verteilerelement (15a), dessen glatte Wand die Blasen des aus den Zuführmitteln stammenden Belüftungsgases durchlässt, die sich in dem inneren Raum (4) in Richtung des unteren Teils (3a) der Wand (3) des Behälters (2) befinden,
**gekennzeichnet durch** die Tatsache, dass:
* er wenigstens einen kombinierten Port Entleerung (6) / unteres Lager (11) mit einem steifen Flansch (16a) umfasst:
- der mit einem Entleerungsdurchgang (17) versehen ist, der auf einer Seite mit dem inneren Raum (4) und auf der anderen mit der Außenseite des Behälters (2) in fluidischer Kommunikation ist,
- der steif und wasserdicht am unteren Teil (3a) der Wand (3) des Behälters (2) um die Entleerungsöffnung (6) befestigt ist, wobei der Entleerungsdurchgang (17) und die Entleerungsöffnung (6) sich in fluidischer Kommunikation befinden,
- der auf der inneren Seite ein inneres Lager (11) trägt, das sich im inneren Raum (4) befindet, der am Entleerungsdurchgang (17) anliegt, ohne die fluidische Kommunikation zwischen dem Entleerungsdurchgang (17) und der Entleerungsöffnung (6) zu verhindern,
* das wenigstens eine erweiterte Verteilerelement (15a) des Belüftungsgases radial vom Port Entleeren (6) / Lager (11) beabstandet ist,
* das wenigstens eine röhrenförmige Zuführelement (14a) des Belüftungsgases sich ab dem erweiterten Verteilerelement (15a) in dem inneren Raum (4) entlang der inneren Seite des unteren Teils (3a) und des lateralen Teils (3b) der Wand (3) des Behälters (2) erstreckt und sich auf der Außenseite des Behälters (2) ab dem - oder der Nachbarschaft des - oberen Teil(s) (3c) der Wand (3) des Behälters (2) erstreckt,
* wenigstens ein Mischorgan (10) vom unteren Teil (3a) der Wand (3) des Behälters (2), dem unteren Lager (11) und dem wenigstens einen erweiterten Verteilerelement (15a) von Belüftungsgas beabstandet ist,
derart, dass die Blasen des Belüftungsgases, die von dem wenigstens einen erweiterten Verteilerelement (15a) des Belüftungsgases ausgegeben werden, in dem Inhalt (C) **durch** eine erste Verteilung in dem unteren Bereich des unteren Raums (4), der am unteren Teil (3a) der Wand (3) des Behälters (2) anliegt, **durch** das wenigstens eine erweiterte Verteilerelement (15a) des Verteilergases und eine zweite Verteilung **durch** das wenigstens eine Mischorgan (10) in dem gesamten inneren Raum (4) des Behälters (2) verteilt werden.

2. Mischer-Behälter (1) gemäß Anspruch 1, **gekennzeichnet durch** die Tatsache, dass die wenigstens eine Welle (8) der Mischmittel (7) entweder mit einem einzigen Lager, dem unteren Lager (11), zusammenwirkt, wobei das Motormittel (9) zum Antreiben der Welle (8) in Rotation sich in Richtung zum unteren Teil (3a) der Wand (3) des Behälters (2) befindet, oder mit zwei Lagern, sein unterer Teil (8a) mit dem unteren Lager (11) und sein oberer Teil (8b) mit einem oberen Lager (11a), das am oberen Teil anliegt.

3. Mischer-Behälter (1) gemäß Anspruch 2, **gekennzeichnet durch** die Tatsache, dass er wenigstens einen kombinierten Port Einführung (5) / oberes Lager (11a) mit einem steifen Flansch (16a) umfasst:
- der mit einem Einführdurchgang des Inhaltes (C) oder von Komponenten des Inhaltes (C) versehen ist, der sich auf einer Seite mit dem inneren Raum (4) und auf der anderen mit der Außenseite des Behälters (2) in fluidischer Kommunikation befindet,
- der steif und abgedichtet am oberen Teil der Wand (3) des Behälters (2) rund um die Einführöffnung befestigt ist, wobei der Einführdurchgang und die Einführöffnung sich in fluidischer Kommunikation befinden,
- der auf der inneren Seite das obere Lager (11a) trägt, das sich im inneren Raum (4) befindet, der am Einführdurchgang anliegt, ohne die fluidische Kommunikation zwischen dem Einführdurchgang und der Einführöffnung zu verhindern.

4. Mischer-Behälter (1) gemäß Anspruch 1 bis 3, **gekennzeichnet durch** die Tatsache, dass die wenigstens eine Welle (8) der Mischmittel (7) sich komplett im inneren Raum (4) befindet, wobei das Motormittel (9) zum Antreiben der Welle (8) in Rotation im Magnetbetrieb funktioniert, wobei eine führende rotierende Scheibe mit magnetischen Polen (9a), die sich an der Außenseite des Behälters (2) befindet, funktional mit einer geführten rotierenden Scheibe mit magnetischen Polen zusammenwirkt, die auf der wenigstens einen Welle (8) in magnetischer Nähe der führenden rotierenden Scheibe (9a) befestigt ist.

5. Mischer-Behälter (1) gemäß Anspruch 1 bis 3, **gekennzeichnet durch** die Tatsache, dass die wenigstens eine Welle (8) der Mischmittel (7) sich zum Teil in dem inneren Raum (4) befindet und zum Teil an der Außenseite des Behälters (2), wobei das Motormittel (9) zum Antreiben der Welle (8) in Rotation im mechanischen Betrieb arbeitet, wobei eine führende rotierende Welle, die sich an der Außenseite des Behälters (2) befindet, funktional mit dem äußeren Teil der wenigstens einen Welle (18) zusammenwirkt.

6. Mischer-Behälter (1) gemäß Anspruch 1 bis 5, **gekennzeichnet durch** die Tatsache, dass die Mischmittel (7) entweder eine einzige dressierte Welle (8) oder mehrere dressierte Wellen (8) mit parallelen Achsen umfassen, die geeignet sind, jedes wenigstens eine Mischorgan (10) in Rotation anzutreiben.

7. Mischer-Behälter (1) gemäß Anspruch 1 bis 6, **gekennzeichnet durch** die Tatsache, dass eine Welle (8) der Mischmittel (7) entweder ein einziges Mischorgan (10), das sich an einer einzigen axialen Stelle auf der Welle (8) befindet, oder mehrere Mischorgane, die sich in einer Vielzahl von axialen Stellen auf der Welle (8) befinden, zu tragen und anzutreiben.

8. Mischer-Behälter (1) gemäß Anspruch 1 bis 7, **gekennzeichnet durch** die Tatsache, dass ein Mischorgan (10) vom unteren Teil (3a) der Wand (3) des Behälters (2) dem unteren Lager (11) und dem wenigstens einen erweiterten Verteilerelement (15a) um eine Entfernung in der Größenordnung von wenigstens einem Viertel der Beabstandung zwischen dem unteren Teil (3a) und dem oberen Teil (3c) der Wand (3) des Behälters (2) beabstandet ist.

9. Mischer-Behälter (1) gemäß Anspruch 1 bis 8, **gekennzeichnet durch** die Tatsache, dass ein Mischorgan (10) vom unteren Teil (3a) der Wand (3) des Behälters (2), vom unteren Lager (11) und dem wenigstens einen erweiterten Verteilerelement (15a) um eine Entfernung in der Größenordnung von wenigstens dem Drittel der Beabstandung zwischen dem unteren Teil (3a) und dem oberen Teil (3c) der Wand (3) des Behälters (2) beabstandet ist.

10. Mischer-Behälter (1) gemäß Anspruch 1 bis 9, **gekennzeichnet durch** die Tatsache, dass das wenigstens eine röhrenförmige Zuführelement (14a) des Belüftungsgases sich in dem inneren Raum (4) erstreckt und dabei angrenzend oder anliegend an der inneren Seite der Wand (3) des Behälters (2) festgehalten wird.

11. Mischer-Behälter (1) gemäß Anspruch 1 bis 10, **gekennzeichnet durch** die Tatsache, dass das wenigstens eine röhrenförmige Zuführelement (14a) des Belüftungsgases wenigstens zum Teil entweder strukturell unterschiedlich von der Wand (3) des Behälters (2) ist und von dieser per Verklebung, Verschweißung oder mittels angesetzten Festhalteteilen festgehalten wird, oder fester Bestandteil der Wand (3) des Behälters (2) ist.

12. Mischer-Behälter (1) gemäß Anspruch 1 bis 11, **gekennzeichnet durch** die Tatsache, dass das wenigstens eine röhrenförmige Zuführelement (14a) des Belüftungsgases die Wand (3) des Behälters (2) **durch** eine abgedichtete Verbindung durchquert.

13. Mischer-Behälter (1) gemäß Anspruch 1 bis 12, **gekennzeichnet durch** die Tatsache, dass das wenigstens eine röhrenförmige Zuführelement (14a) des Belüftungsgases die Wand (3) des Behälters (2) im oberen Teil (3c) durchquert.

14. Mischer-Behälter (1) gemäß Anspruch 1 bis 13, **gekennzeichnet durch** die Tatsache, 1 dass das wenigstens eine erweiterte Verteilerelement (15a) des Belüftungsgases angrenzend oder anliegend an der inneren Seite des unteren Teils (3a) der Wand (3) des Behälters (2) festgehalten wird.

15. Mischer-Behälter (1) gemäß Anspruch 1 bis 14, **gekennzeichnet durch** die Tatsache, dass das wenigstens eine erweiterte Verteilerelement (15a) des Belüftungsgases wenigstens zum Teil strukturell unterschiedlich von der Wand (3) des Behälters (2) ist und **durch** sie per Verklebung, Verschweißung oder mittels angesetzter Festhalteteile festgehalten wird.

16. Mischer-Behälter (1) gemäß Anspruch 1 bis 15, **gekennzeichnet durch** die Tatsache, dass das wenigstens eine erweiterte Verteilerelement (15a) des Belüftungsgases wenigstens zum Teil strukturell fester Bestandteil der Wand (3) des Behälters (2) ist.

17. Mischer-Behälter (1) gemäß Anspruch 1 bis 16, **gekennzeichnet durch** die Tatsache, dass das wenigstens eine röhrenförmige Zuführelement (14a) sich von oben nach unten ab der Außenseite bis zur Innenseite des Behälters (2) und des erweiterten Verteilerelements (15a) des Belüftungsgases mit dem wenigstens einen röhrenförmigen Zuführelement (14a) in Entsprechung ist.

18. Mischer-Behälter (1) gemäß Anspruch 1 bis 17, **gekennzeichnet durch** die Tatsache, dass das wenigstens eine erweiterte Verteilerelement (15a) des Belüftungsgases eine Wand (3) umfasst, die mit einer Vielzahl von verteilten Löchern versehen ist, die für den Durchgang der Blasen des Belüftungsgases geeignet sind, das aus den Zuführmitteln (14) stammt.

19. Mischer-Behälter (1) gemäß Anspruch 18, **gekennzeichnet durch** die Tatsache, dass die Vielzahl von Löchern, die für den Durchgang der Blasen des Belüftungsgases geeignet sind, das aus den Zuführmitteln (14) stammt, mit unterschiedlichen Neigungsachsen auf der Vertikalen ausgerichtet ist.

20. Mischer-Behälter (1) gemäß Anspruch 18 und 19, **gekennzeichnet durch** die Tatsache, dass die Löcher der Vielzahl von Löchern entweder dieselbe Größe oder unterschiedliche Größen haben.

21. Mischer-Behälter (1) gemäß Anspruch 1 bis 20, **gekennzeichnet durch** die Tatsache, dass das wenigstens eine erweiterte Verteilerelement (15a) des Belüftungsgases im geraden Querschnitt eine kreisförmige oder pseudo-kreisförmige oder elliptische oder pseudoelliptische Form hat.

22. Mischer-Behälter (1) gemäß Anspruch 1 bis 21, **gekennzeichnet durch** die Tatsache, dass das wenigstens eine erweiterte Verteilerelement (15a) des Belüftungsgases wenigstens einen vollständigen, auf sich selbst geschlossenen Ring in kreisförmiger Kommunikation oder nicht und / oder einen unvollständigen, im Verhältnis zu sich selbst offenen Ring, der insbesondere eine zwischen rund 180° und 270° inbegriffene Winkelöffnung hat, umfasst.

23. Mischer-Behälter (1) gemäß Anspruch 1 bis 22, **gekennzeichnet durch** die Tatsache, dass die Belüftungsmittel (13) eine einzige Gruppe von Zuführmitteln (14) des Belüftungsgases und Verteilermittel (5) des Belüftungsgases oder mehrere unterschiedliche Gruppen von Zuführmitteln eines oder mehrerer Belüftungsgas(e) und Verteilermittel (15) des oder der Belüftungsgas(e) umfassen.

24. Mischer-Behälter (1) gemäß Anspruch 23, **gekennzeichnet durch** die Tatsache, dass eine Gruppe von Belüftungsmitteln (13) ein einziges röhrenförmiges Zuführelement (14a) des Belüftungsgases umfasst, das mit einem einzigen erweiterten Verteilerelement (15a) des Belüftungsgasees kommuniziert, oder ein einziges röhrenförmiges Zuführelement (14a) des Belüftungsgases, das mit mehreren erweiterten Verteilerelementen (15a) des Belüftungsgases kommuniziert, oder mehrere röhrenförmige Zuführmittel (14a) des Belüftungsgases, die mit einem einzigen erweiterten Verteilerelement (15a) des Belüftungsgases kommuniziert oder mehrere röhrenförmige Zuführelemente (14a) des Belüftungsgases, die mit mehreren erweiterten Verteilerelementen (15a) des Belüftungsgases kommunizieren.

25. Mischer-Behälter (1) gemäß Anspruch 1 bis 24, in dem Fall, in dem er mehrere unterschiedliche erweiterte Verteilerelemente (15a) des Belüftungsgases umfasst, **gekennzeichnet durch** die Tatsache, dass wenigstens einige der mehreren erweiterten Verteilerelemente (15a) des Belüftungsgases sich in einer Vielzahl von radialen Stellen in dem inneren Raum (4) in Richtung zum unteren Teil (3a) des Behälters (2) befinden, insbesondere radial vom Port Entleeren (6) / Lager (11) bis zur Nachbarschaft zum lateralen Teil (3b) der Wand (3) des Behälters (2) beabstandet sind.

26. Mischer-Behälter (1) gemäß Anspruch 1 bis 25, **gekennzeichnet durch** die Tatsache, dass ein erweitertes Verteilerelement (15a) des Belüftungsgases radial vom Port Entleeren (6) / Lager (11) um eine Entfernung in der Größenordnung von wenigstens einem Fünftel des Durchmessers des unteren Teiles (3a) der Wand (3) des Behälters (2) beabstandet ist.

27. Mischer-Behälter (1) gemäß Anspruch 1 bis 26, **gekennzeichnet durch** die Tatsache, dass unter dem unteren Teil (3a) der Wand (3) des Behälters (2) nur die Entleerung (6) und ggf. das Motormittel (9) zum Antreiben der Mischmittel (7), wenn es im unteren Teil vorgesehen ist, hervorsteht.

28. Mischer-Behälter (1) gemäß Anspruch 1 bis 27, **gekennzeichnet durch** die Tatsache, dass er ebenfalls einen oder mehrere Austragsport(s) des Gases umfasst, der / die mit wenigstens einer Austragsöffnung zusammenwirkt / zusammenwirken, die im oberen Teil der Wand (3) des Behälters (2) ausgespart ist und mit einem Anti-Rückschlagventil, das das Einführen von unerwünschten oder nicht gewünschten Fluiden oder Kontaminanten in den Behälter (2) verhindert, und / oder einem oder mehreren Einführ- (5), Entleerungs- (6), Montage-port(s), versehen ist.

29. Mischer-Behälter (1) gemäß Anspruch 1 bis 28, **gekennzeichnet durch** die Tatsache, dass der Behälter (2) eine hohe Kapazität aufweist, die bis zu 5.000 Litern gehen kann.

30. Mischer-Behälter (1) gemäß Anspruch 1 bis 29, **gekennzeichnet durch** die Tatsache, dass er ebenfalls eine äußere steife Vorrichtung (18) zum Fixieren des mit seinem Inhalt (C) befüllten Behälters (2) mit einer Bodenwand (19), einer peripherischen Wand (20) und einer oberen Öffnung (21) umfasst, die eine Hauptaufnahme begrenzt, in der abnehmbar der flexible Behälter (2) platziert ist, dessen unterer Teil auf der Bodenwand (19) ruht und dessen lateraler Teil gegen die peripherische Wand (20) angewendet wird, wenn der Behälter (2) mit seinem Inhalt (C) gefüllt ist.

31. Mischer-Behälter (1) gemäß Anspruch 30, **gekennzeichnet durch** die Tatsache, dass die äußere steife Vorrichtung (18) zum Fixieren ebenfalls eine sekundäre Aufnahme (22) unterhalb der Bodenwand (19) der Aufnahme und des Schutzes der Entleerung (6) und ggf. des Motormittels (9) zum Antreiben der Mischmittel (7) umfasst, wenn dies im unteren Teil vorgesehen ist.

32. Mischer-Behälter (1) gemäß Anspruch 30 und 31, **gekennzeichnet durch** die Tatsache, dass die äußere steife Vorrichtung (18) zum Fixieren ebenfalls Heizmittel umfasst und der flexible Behälter (2) aus einem Material ist, das eine bestimmte thermische Leitfähigkeit derart aufweist, dass die Umsetzung der Heizmittel das Heizen des Inhaltes (C) erlaubt; und ggf. Kontrollmittel der Temperatur des Behälters (2) und Steuermittel der Heizmittel.

33. Mischer-Behälter (1) gemäß Anspruch 30 bis 32, **gekennzeichnet durch** die Tatsache, dass der Behälter (2) sich in drei extremen Zuständen befinden kann: einem demontierten Zustand der äußeren steifen Vorrichtung (18) zum Fixieren, in dem der Behälter (2) abgeflacht auf sich selbst angeordnet sein kann, einem mit der äußeren steifen Vorrichtung (18) zum Fixieren zusammengebauten Zustand, in dem der Behälter (2) ohne Inhalt (C) in der Hauptaufnahme der Vorrichtung zum Fixieren (18) angeordnet ist, indem er auf der Bodenwand (19) ruht, und einem mit der äußeren steifen Vorrichtung (18) zum Fixieren zusammengebauten Zustand, in dem der mit seinem Inhalt (C) gefüllte Behälter (2) in der Hauptaufnahme der Vorrichtung zum Fixieren (18) angeordnet ist und auf der Bodenwand (19) ruht und sich gegen die peripherische Wand (20) angewendet befindet.

34. Mischer-Behälter (1) gemäß Anspruch 1 bis 33, **gekennzeichnet durch** die Tatsache, dass man dort eine Bioreaktion realisiert, wobei der Mischer-Behälter (1) ein Bioreaktor ist.

35. Umsetzungsverfahren eines Mischer-Behälters (1) gemäß Anspruch 1 bis 34, in dem:
* man über einen derartigen Mischer-Behälter (1) verfügt, dessen Entleerungsport (6) verschlossen ist,
* man über den Inhalt (C) oder Komponenten des Inhalts (C) verfügt, die dazu bestimmt sind, in dem Behälter (2) des Mischer-Behälters (1) aufgenommen und gemischt zu werden,
* man den Inhalt (C) oder Komponenten des Inhaltes (C) in den Behälter (2) einführt,
* man die Mischmittel (7) umsetzt, um den Inhalt (C) des Behälters (2) zu schütteln,
* man die Belüftungsmittel (13) umsetzt, um dem Inhalt (C) eine bestimmte Menge von Belüftungsgas zuzuführen, wobei die Belüftung und die Mischung wenigstens zum Teil gleichzeitig realisiert werden und
* man die Blasen des Belüftungsgases von dem wenigstens einen erweiterten Verteilerelement (15a) des Belüftungsgases verteilt und man sie in dem Inhalt (C) durch eine erste Verteilung in dem unteren Bereich des inneren Bereichs (4), der an dem unteren Teil (3a) der Wand (3) des Behälters (2) anliegt, durch das wenigstens eine erweiterte Verteilerelement (15a) des Belüftungsgases und eine zweite Verteilung durch das wenigstens eine Mischorgan (10) in dem gesamten inneren Raum (4) des Behälters (2) verteilt.

36. Verfahren gemäß Anspruch 35, **gekennzeichnet durch** die Tatsache, dass man zunächst in den Behälter (2) eine Komponente oder einen Teil der Komponenten des Inhalts (C) einführt, man die Mischmittel (7) und die Belüftungsmittel (13) umsetzt und man eine bestimmte Menge Belüftungsgas einführt, um sie an den Inhalt (C) auszugeben, und man in den Behälter (2) die restliche Komponente oder restlichen Komponenten des Inhaltes (C) einführt.

37. Verfahren gemäß Anspruch 35 und 36, **gekennzeichnet durch** die Tatsache, dass:
* man von einem Mischer-Behälter (1) ausgeht, dessen Behälter (2) von einer äußeren steifen Vorrichtung (18) zum Fixieren ohne Inhalt (C) demontiert ist und abgeflacht auf sich selbst angeordnet ist,
* man den Behälter (2) mit der äußeren steifen Vorrichtung (18) zum Fixieren montiert, indem er in der Hauptaufnahme desselben angeordnet wird und dabei auf seiner Bodenwand (19) ruht,
* man dann in den Behälter (2) den Inhalt (C) oder die Komponenten des Inhaltes (C) einführt.

38. Verfahren gemäß Anspruch 35 bis 37, **gekennzeichnet durch** die Tatsache, dass man unter dem unteren Teil (3a) der Wand (3) des Behälters (2) die Entleerung (6), das Motormittel (9) zum Antreiben der Mischmittel (7) anordnet, wenn es im unteren Teil vorgesehen ist.
